# EUROPEAN PATENT SPECIFICATION

(11) **EP 3 075 845 B1**
(45) Date of publication and mention of the grant of the patent: **05.06.2019**
(21) Application number: 14865114.4
(22) Date of filing: 27.11.2014
(51) Int. Cl.: C12N 5/0775, A61K 35/28, C12N 15/113, C12N 15/117, G01N 33/53

(54) **NOVEL MESENCHYMAL STEM CELL SURFACE MARKER**
NEUARTIGER OBERFLÄCHENMARKER FÜR MESENCHYMALE STAMMZELLEN
NOUVEAU MARQUEUR DE SURFACE DE CELLULES SOUCHES MÉSENCHYMATEUSES

(30) Priority: 27.11.2013 ES 201331730
(43) Date of publication of application: 05.10.2016
(73) Proprietor: Fundación Pública Andaluza Progreso Y Salud, 41092 Sevilla (ES)
(72) Inventor: ANDERSON, Per, S-41092 Sevilla (SE); MARTÍN MOLINA, Francisco, S-41092 Sevilla (ES); CARRILLO GÁLVES,Ana, S-41092 Sevilla (ES); COBO PULIDO, Marién, S-41092 Sevilla (ES)
(74) Representative: Hoffmann Eitle
(86) International application number: PCT/ES2014/070874
(87) International publication number: WO 2015/079091

(56) References cited:
- WO-A2-2006/103639
- WO-A2-2009/114860
- US-A1- 2011 300 119
- WANG RUI ET AL: "Identification of a Regulatory T Cell Specific Cell Surface Molecule that Mediates Suppressive Signals and Induces Foxp3 Expression", PLOS ONE, PUBLIC LIBRARY OF SCIENCE, US, vol. 3, no. 7, 16 July 2008 (2008-07-16), pages e2705-1, XP009125579, ISSN: 1932-6203
- CARRILLO-GALVEZ, A. B. ET AL.: 'Mesenchymal stromal cells express GARP/LRRC32 on their surface: effects on their biology and immunomodulatory capacity.' STEM CELLS (DAYTON, OHIO) UNITED STATES vol. 33, no. 1, January 2015, pages 183 - 195, XP055346023
- DATABASE GENBANK [Online] 13 August 2013 'HOMO SAPIENS CHROMOSOME 11, GRCH37.P13 PRIMARY ASSEMBLY', XP008183719 Retrieved from NCBI Database accession no. NC 000011
- BARBET R. ET AL.: 'Comparison of Gene Expression in Human Embryonic Stem Cells, hESC-Derived Mesenchymal Stem Cells and Human Mesenchymal Stem Cells.' STEM CELLS INTERNATIONAL UNITED STATES vol. 2011, 2011, page 368192, XP055240741
- WANG, L. ET AL.: 'Expression of GARP selectively identifies activated human FOXP3 regulatory T cells.' PROC. NATL. ACAD. SCI. USA vol. 106, no. 32, 11 August 2009, pages 13439 - 13444, XP055147094

## Description

The present invention is comprised in the field of cell therapy and regenerative medicine, and relates to a method for the isolation and/or identification of mesenchymal stem cells based on the detection of the GARP (glycoprotein-A repetitions predominant protein) surface marker. Also described are mesenchymal stem cells expressing said marker and uses thereof.

### INTRODUCTION

Mesenchymal stromal cells (MSCs) are non-hematopoietic multipotent cells, with self-renewal capacity and present in virtually all tissues. MSCs secrete a large amount of growth factors and have important immunomodulatory properties, making them a promising tool for therapies in autoimmune diseases. The injection of MSCs *ex vivo* effectively reduces autoimmune/inflammatory diseases in animal models and many efforts are being made to achieve these results in human therapies (Caplan and Correa, 2011. Cell Stem Cell 9,11-15). Recent data suggests that therapy with MSCs in humans is safe and promising; however, several clinical trials have not shown any significant benefit (Galipeau, 2013. Cytotherapy 15, 2-8; Herreros et al., 2012. Dis. Colon Rectum 55, 762-772). A possible explanation for these disappointing results is the heterogeneity of MSC preparations used, specifically with respect to their differentiation, homing or recruitment and their immunosuppressive capacity. This is because the MSC preparations used in many of the mentioned clinical studies contain, in practice, in addition to undifferentiated MSC cells, different types of cells with clear biochemical and phenotypic differences representing different types of mesodermal progenitors in different stages of differentiation. This heterogeneity has been proven by several studies, among them the study conducted by Tremain *et al.* (Tremain et al., 2001. Stem Cells 19, 408-418). This group performed the transcriptional analysis of individual cells derived from MSC colonies, observing the simultaneous expression of transcripts that are characteristic of various mesenchymal cell lineages, indicating that the MSC preparations are not homogenous.

This problem is largely due to the fact that right now there is no specific MSC marker, and therefore the isolation of said cells basically depends on their adherence to plastic or the lack thereof. Additionally, the low frequency at which cells of this type are found adds to the problem. Therefore, their low frequency and the lack of specific markers make these cells difficult to identity, select or isolate and study *in vivo.* Furthermore, the characteristic markers used *in vitro* can be induced by culturing and are not expressed in MSC/MSC progenitors *in vivo.* Therefore, an important discovery would be the identification of an MSC marker that allowed the easy isolation of a population of MSCs that has a potent self-renewal and multipotency capacity and that can be used for therapeutic purposes. It has recently been proven that nestin marks a primitive population of MSCs in the bone marrow of mice (Mendez-Ferrer et al., 2010. Nature 466, 829-834). However, nestin is an intracellular protein and cannot be used for the selection of these cells.

There is therefore a need to identify MSC surface proteins actively participating in the therapeutic activity of said cells. These surface proteins would be used not only for identifying and isolating subpopulations of MSCs but also for enhancing their therapeutic activity.

### BRIEF DESCRIPTION OF THE INVENTION

In a first aspect, the present invention relates to the *in vitro* use of the GARP protein (glycoprotein-A repetitions predominant protein) for the identification and optionally isolation of activated mesenchymal stem cells.

A preferred embodiment of the first aspect of the invention relates to an *in vitro* method for the identification and/or isolation of a mesenchymal stem cell, preferably an activated mesenchymal stem cell, from a biological sample comprising said mesenchymal stem cells, comprising the detection of the GARP protein on the surface of said cell and the isolation of said MSCs based on the expression of said protein on the surface thereof (LRRC32).

Also described is an isolated mesenchymal stem cell, hereinafter mesenchymal stem cell, that can be obtained by the method of the first aspect of the invention comprising the detection of GARP (glycoprotein-A repetitions predominant protein) on the surface of said cell and the isolation of said cell based on the expression of said protein.

Also described is an isolated cell population, hereinafter isolated cell population, comprising at least one mesenchymal stem cell. The isolated cell population is a substantially pure cell population comprising at least 50%, 60%, 70%, 80%, 85%, 90%, 91%, 92%, 93%, 94%, 95%, 96%, 97%, 98% or 99% of MSC type cells isolated by a method comprising the detection of GARP or obtained from one or more MSC cells isolated by a method comprising the detection of GARP.

Also described is a GARP expression inducing agent in MSC cells, selected from the list consisting of:
a) an organic molecule,
b) a DNA or RNA molecule,
c) an antisense oligonucleotide,
d) an antibody, or
e) a ribozyme
or any combination thereof, for increasing the immunomodulatory effect of the MSCs.

Also described is an isolated RNA or DNA polynucleotide, hereinafter polynucleotide, capable of being translated into an amino acid sequence comprising a peptide having an identity of at least 80%, more preferably 85%, 90%, and even more preferably 95% with the amino acid sequence of the GARP protein (SEQ ID NO: 1). More preferably, the isolated RNA or DNA polynucleotide is capable of being translated into an amino acid sequence comprising a peptide having an identity of at least 60%, preferably 70%, 80%, 90%, 95%, 98%, or even more preferably 99%, with the amino acid sequence of the GARP protein (SEQ ID NO: 1)

Also described is a DNA or RNA gene construct, hereinafter gene construct, comprising at least one of the following types of sequences:
a) nucleotide sequence comprising at least one polynucleotide as described above, for *in vitro* or *in vivo* transcription;
b) nucleotide sequence corresponding to a gene expression vector or system comprising a polynucleotide as described above, operatively linked with at least one promoter directing the transcription of said nucleotide sequence, and with other sequences necessary or suitable for the transcription and the suitable regulation thereof in time and place.

Also described is an isolated cell, hereinafter second isolated cell, comprising the isolated RNA or DNA polynucleotide, or the gene construct as described above. Preferably, the second cell is a mesenchymal stem cell of a mammal, and more preferably it is a human mesenchymal stem cell.

Also described is a composition, hereinafter composition, comprising the mesenchymal stem cell, the cell population or the substantially pure cell population, the isolated RNA or DNA polynucleotide, the gene construct or the second isolated cell, all as described above.

Also described is the use of the mesenchymal stem cell, the cell population or the substantially pure cell population, the isolated RNA or DNA polynucleotide, the gene construct or the second isolated cell, all as described above, in the production of a medicinal product. Alternatively, the mesenchymal stem cell, the cell population or the substantially pure cell population, the isolated RNA or DNA polynucleotide, the gene construct or the second isolated cell, all as described above, may be used in therapy.

Also described is the use of the isolated mesenchymal stem cell, the cell population or the substantially pure cell population, the isolated RNA or DNA polynucleotide, the gene construct or the second isolated cell, all as described above, in the production of a medicinal product for tissue and organ repair or regeneration. Alternatively, it is described the mesenchymal stem cell, the cell population or the substantially pure cell population, the isolated RNA or DNA polynucleotide, the gene construct or the second isolated cell, all as described above, for use in tissue and organ repair or regeneration.

Also described is the use of the isolated mesenchymal stem cell, the cell population or the substantially pure cell population, the isolated RNA or DNA polynucleotide, the gene construct or the second isolated cell, all as described above, in the production of a medicinal product for immunomodulation. Alternatively, it is described the mesenchymal stem cell, the cell population or the substantially pure cell population, the isolated RNA or DNA polynucleotide, the gene construct or the second isolated cell, all as described above, for use as immunomodulator.

Also described is the use of the isolated mesenchymal stem cell the cell population or the substantially pure cell population, the isolated RNA or DNA polynucleotide, the gene construct or the second isolated cell, all as described above, in the production of a medicinal product for the treatment of immune diseases or disorders. Alternatively, it is described the mesenchymal stem cell, the cell population or the substantially pure cell population, the isolated RNA or DNA polynucleotide, the gene construct or the second isolated cell, all as described above, for use in the treatment of immune diseases or disorders.

Preferably, the immune diseases or disorders are selected from the list consisting of rheumatoid arthritis, Behçet's disease, amyotrophic lateral sclerosis, multiple sclerosis and Devic's disease, spondyloarthropathy, fibromyalgia, rheumatic fever, Wegener's granulomatosis, systemic lupus erythematosus, Hughes syndrome or antiphospholipid syndrome, polymyositis, dermatomyositis, chronic inflammatory demyelinating polyneuropathy, psoriasis, immune thrombocytopenic purpura, sarcoidosis, chronic fatigue syndrome, Guillain-Barre syndrome, Sjögren's syndrome, systemic vasculitis or vitiligo.

### DESCRIPTION OF THE DRAWINGS

### DRAWINGS

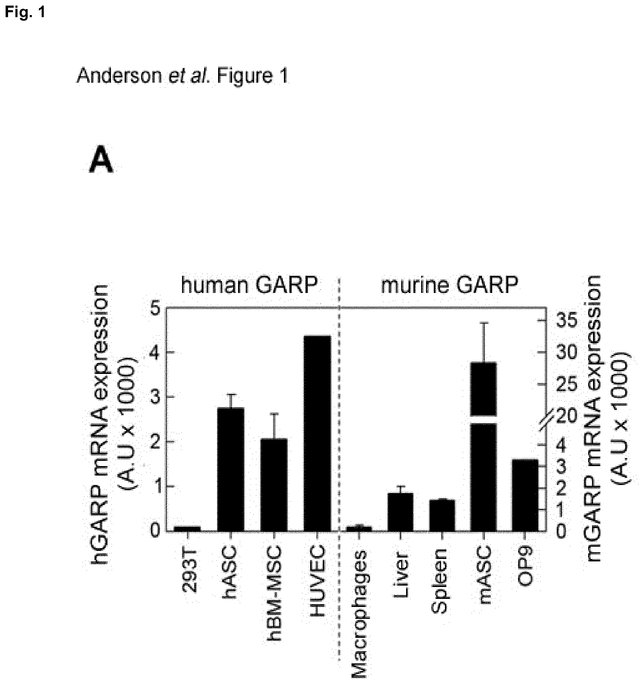

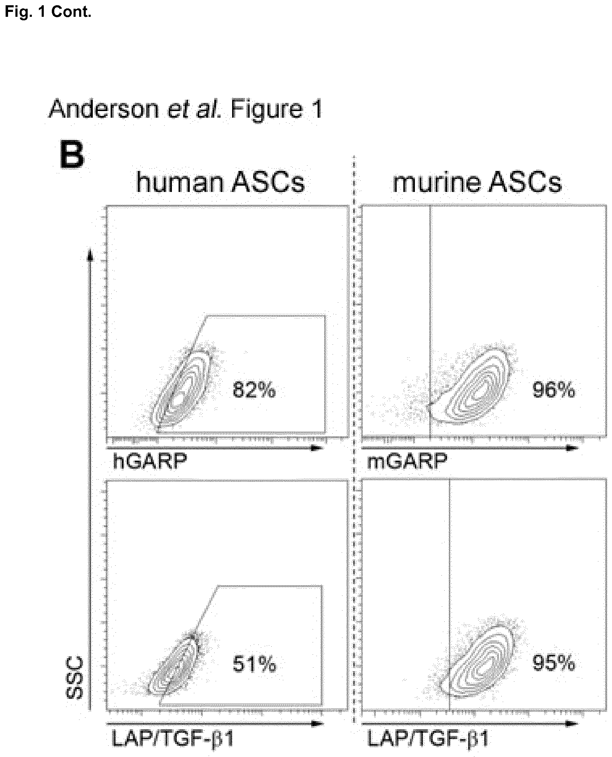

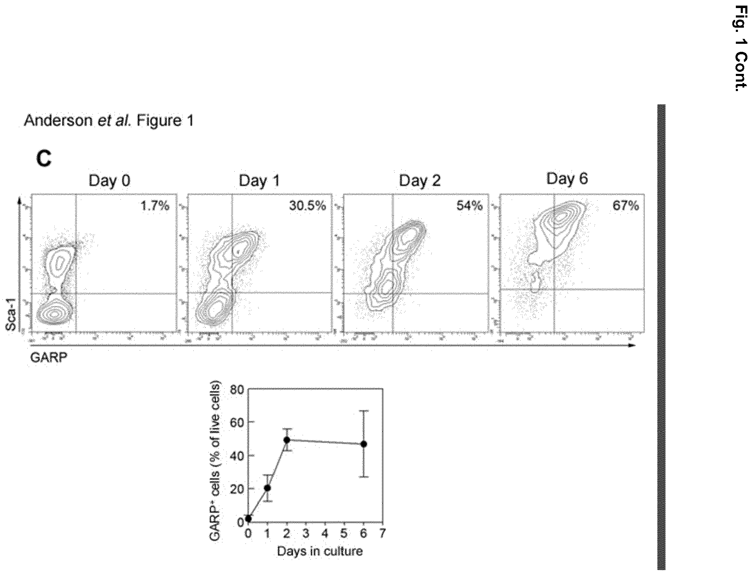

**Figure 1****:** Murine and human MSCs express the GARP protein on their surface.
   (A) Sub-confluent cultures of mASCs (adipose-derived stem cells (ASCs)) collected with EDTA and stained for LAP/TGF-β1 expression. (B) Total RNA extracted from macrophages derived from murine bone marrow, liver and spleen of mice, mASCs and the murine mesenchymal stromal cell line OP9, reverse transcription being performed and GARP expression being analyzed by means of qPCR. mASCs collected with EDTA and stained with (C) for GARP expression or (D) co-stained for GARP and LAP/TGF-β1 expression and analyzed by flow cytometry. (E) qPCR analysis of GARP in human MSCs, 293T and HUVEC cells. (F) GARP staining in hASCs. (B) The cell suspension prepared from visceral and subcutaneous fat deposits stained with an anti-GARP antibody, before and 1, 2 and 6 days after seeding in 6-well plates.

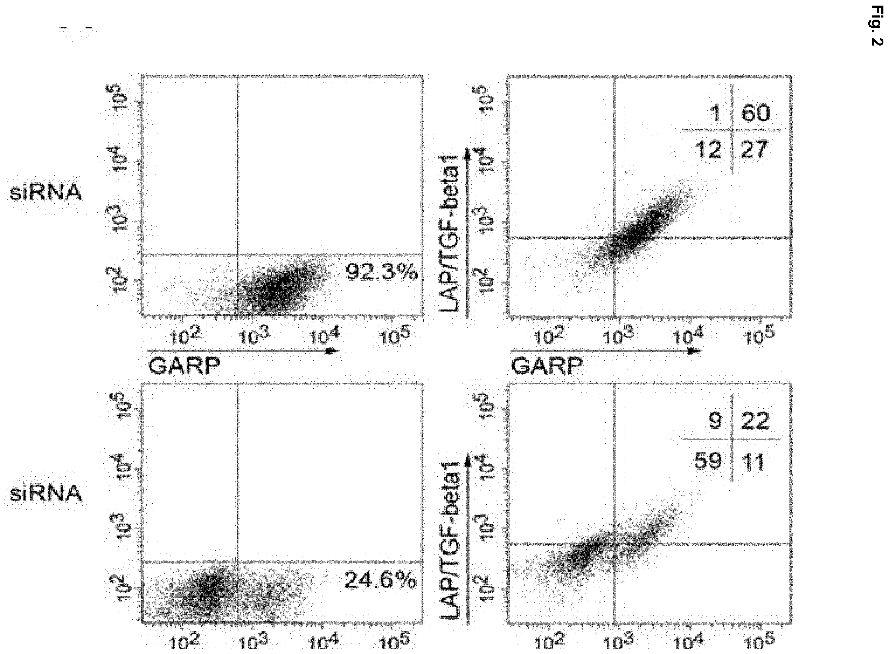

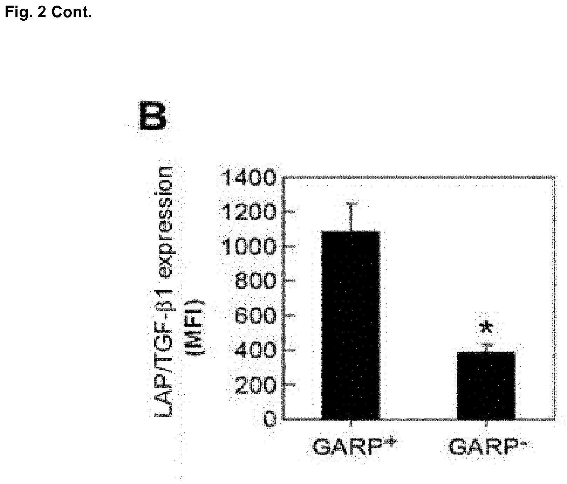

**Figure 2****:** GARP binds TGF-β1 to the ASC surface
   (A) Murine ASCs transduced with lentiviral vectors expressing a non-specific siRNA (NS) or specific GARP siRNA and stained for GARP (left panels) or GARP and LAP/TGF-β1 (right panels). A representative experiment out of every four experiments is shown. (B) LAP/TGF-β1 (MFI) expression levels of GARP + and GARP-ASCs measured after silencing in a flow cytometer. The results are shown as the mean (SEM) of 4-5 independent experiments. * *p*=0.05.

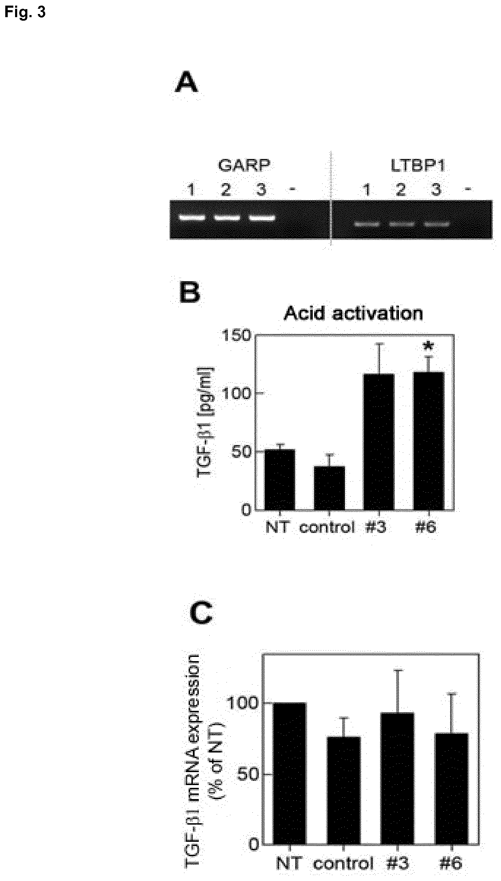

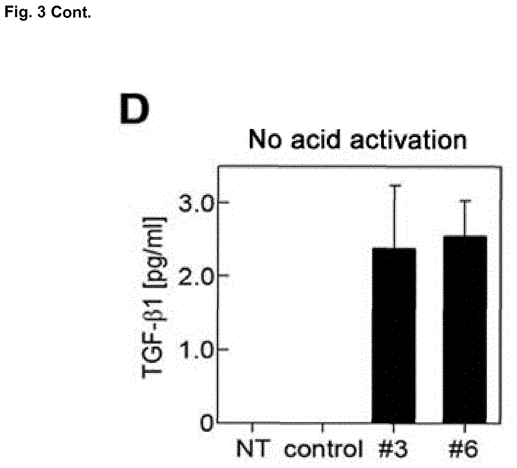

**Figure 3****:** GARP controls TGF-β1 secretion
   (A) GARP and LTBP1 expression in ASCs, not transduced, evaluated by means of qPCR. (B) mASCs, not transduced, control siRNA (control) and specific GARP siRNA (#3 and #6), transduced, cultured for 48 hours and TGF-β1 levels analyzed in activated supernatants. (C) Expression of TGFbeta1 transcripts in ASCs NT, CTRL, #3 and #6 analyzed by qPCR. (D) mASCs, not transduced, control siRNA (control) and specific GARP siRNA (#3 and #6), transduced, cultured for 48 hours and TGF-β1 levels analyzed in non-activated supernatants. The results are shown as the mean (SEM) of 3 independent experiments.

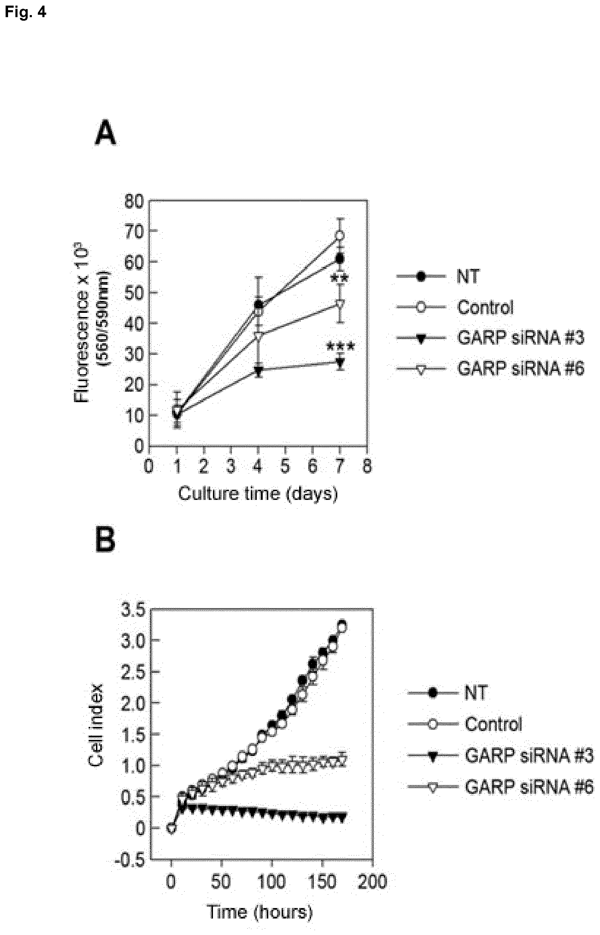

**Figure 4****:** GARP silencing affects mASC proliferation
   mASCs, not transduced, control siRNA (control) and specific GARP siRNA (#3 and #6), transduced, are added (800 cells/well) to (A) 96-well plates and after 1, 4 and 7 days Celltiter-blue is added to the cultures and incubated for 4 hours at 37°C. Fluorescence is measured at 560 nm. (B) Cells are added (800 cells/well) to 16-well E-plates and proliferation is measured in real time in an xCELLigence real time cell analysis system. **p*<0.05.

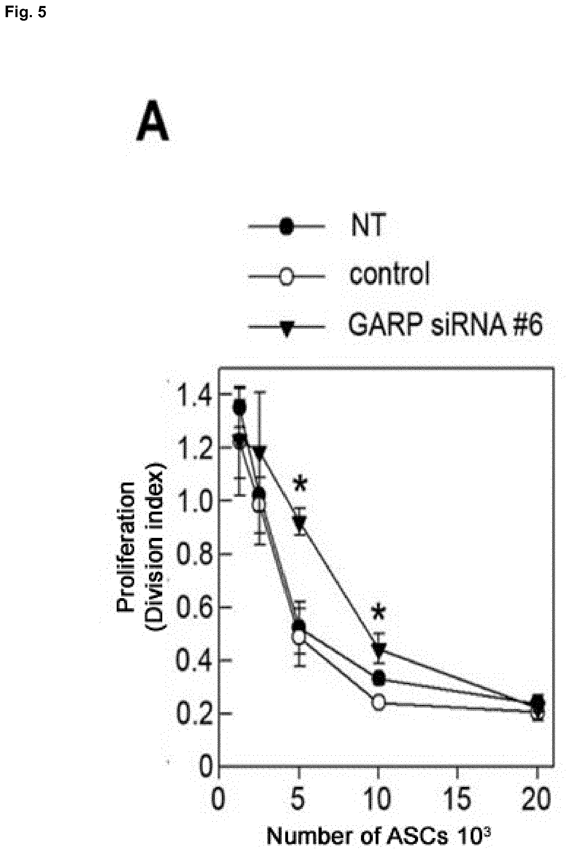

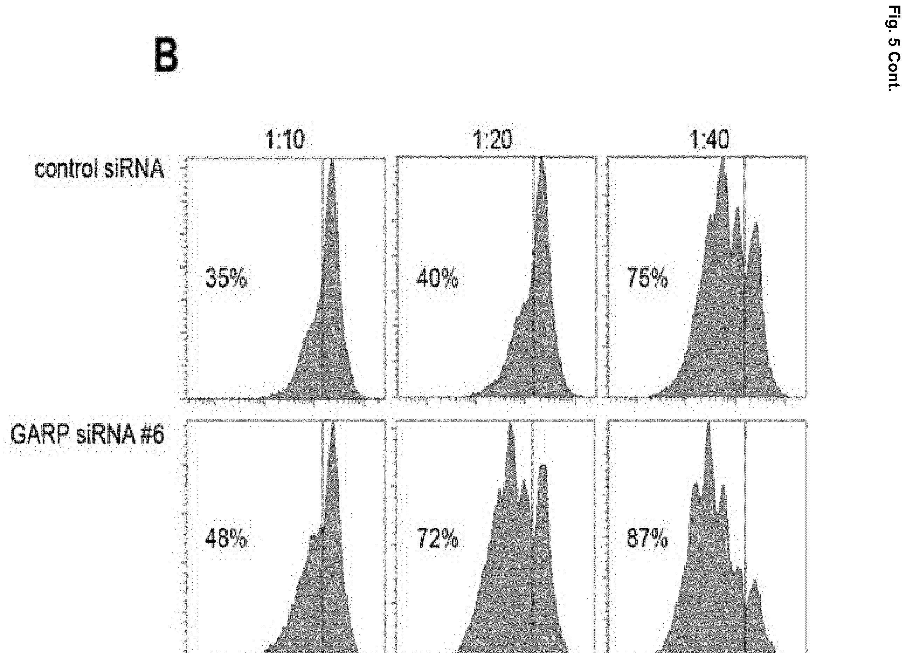

**Figure** 5. Role of GARP in immunomodulation mediated by ASC, mASCs, not transduced, control siRNA (control) and specific GARP siRNA (#3 and #6), transduced, treated with mitomycin C and seeded in different numbers in 96-well plates. Balb/c splenocytes (200,000 cells/well) labeled with CFSE and added to the ASC cultures and activated using antiCD3 (1 µg/ml). After 72 hours, the cells were collected and the dilution of CFSE in CD4+ T-cells was analyzed in a FACS Canto II cytometer using FlowJo software. The results are depicted as (A) division index and (B) histograms representative of CFSE-dilution in CD4+ cells. The results are shown as the mean (SEM) of 3 independent experiments. **p* = 0.05.
**Figure 6****.** Phenotypic characterization and differentiation capacity of murine ASCs.
   (A) Murine ASCs stained for a marker panel used for identifying MSCs and analyzed in a flow cytometer. (B). MSCs induced to differentiate into adipocytes (Oil Red O staining), osteocytes (alizarin red staining) and chondrocytes (alcian blue).
**Figure 7****.** LAP/TGF-β1 staining sensitivity for TrypLE (A) murine ASCs harvested with TrypLE (left panels) or EDTA (right panels) and stained for GARP and LAP/TGF-β1 and analyzed in a flow cytometer.
**Figure 8****.** GARP expression in murine MSCs
   Murine ASCs isolated from visceral fat (omentum), subcutaneous fat (inguinal fat), MSCs derived from bone marrow and cells OP9 are stained for GARP and analyzed by flow cytometry.
**Figure 9****.** mASCs Transduction with GARP siRNA lentiviral vectors
   Genomic DNA isolated from control, mASCs #3 and #6, transduced, one week after transduction and the mean number of vector integrations in the genome measured by qPCR, described in materials and methods. (B) GARP silencing in ASCs evaluated by means of qPCR (left panel) and flow cytometry (right panel).

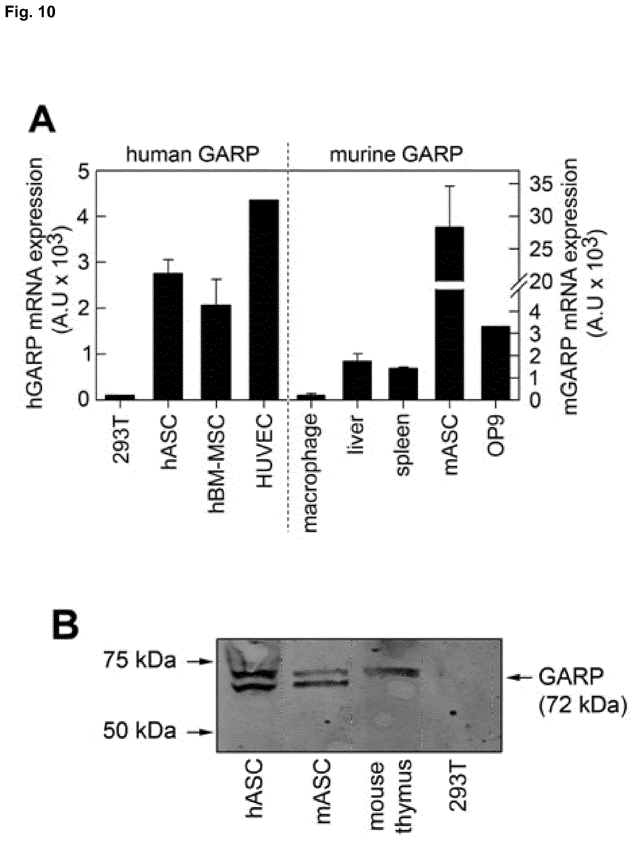

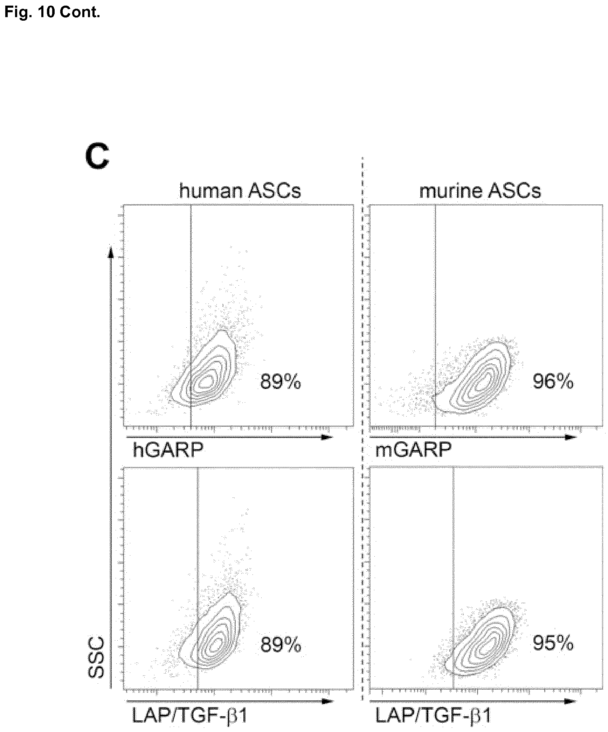

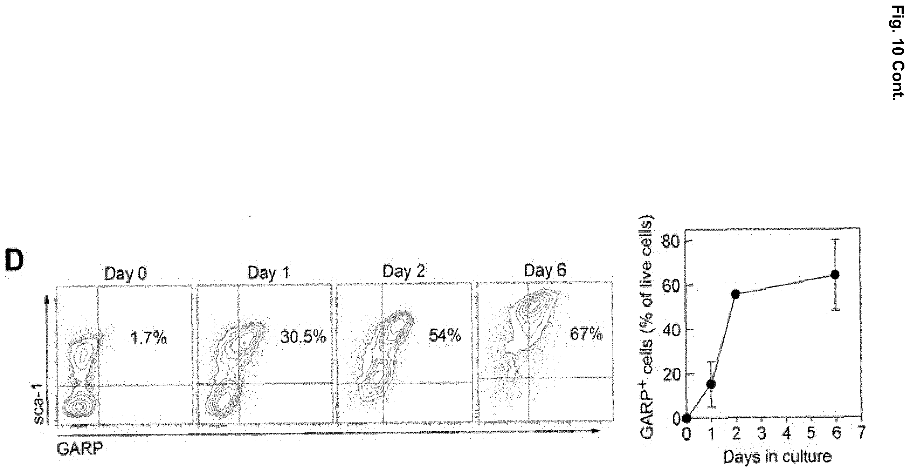

**Figure 10****.** Murine and human MSCs express GARP and LAP/TGF-β1 on their surface
   Analysis of GARP expression in human MSCs (right) and mice (right) by means of RT-qPCR. Total RNA of human mesenchymal cells derived from fat (hASCs) or bone marrow (BM-MSCs), 293T, HUVECs, macrophages derived from murine bone marrow, liver and spleen of mice, mASCs and the murine mesenchymal stromal cell line OP9 was subjected to reverse transcription and GARP expression was analyzed by means of qPCR. (B) Protein extracts of hASCs, mASCs, murine thymocytes and 293T cells were analyzed by means of Western blot using an anti-murine/human GARP antibody. (C) mASCs (right) and hASCs (left) collected with EDTA stained for detecting GARP expression (top) or LAP/TGF-β1 (bottom) and analyzed by flow cytometry. (D) The cell suspension prepared from visceral and subcutaneous fat deposits stained with an anti-GARP antibody, before and 1, 2 and 6 days after seeding in 6-well plates. A and D are shown as the average (SEM) of 3 independent experiments; B and C are shown as representative experiments out of more than 3 independent experiments.

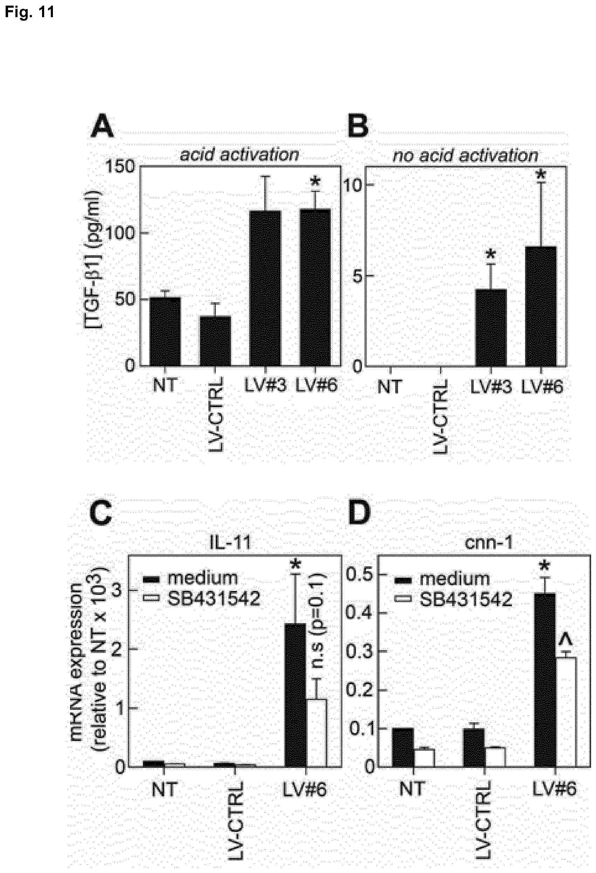

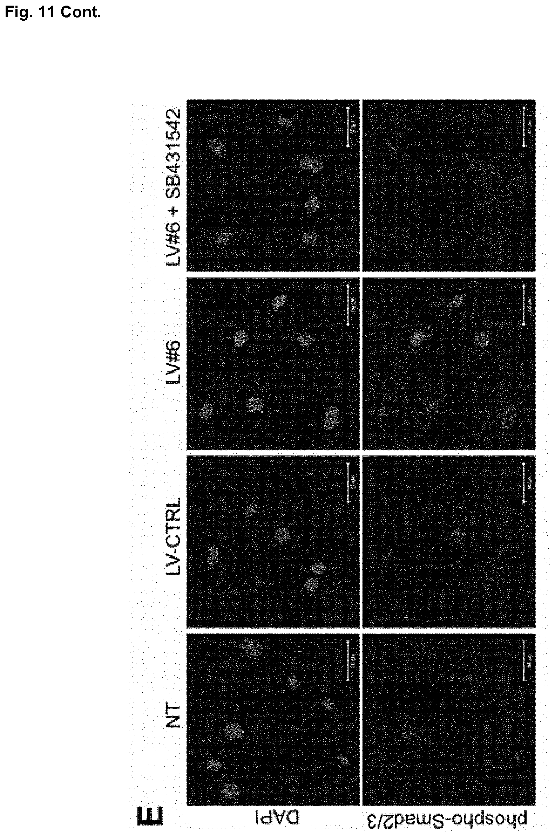

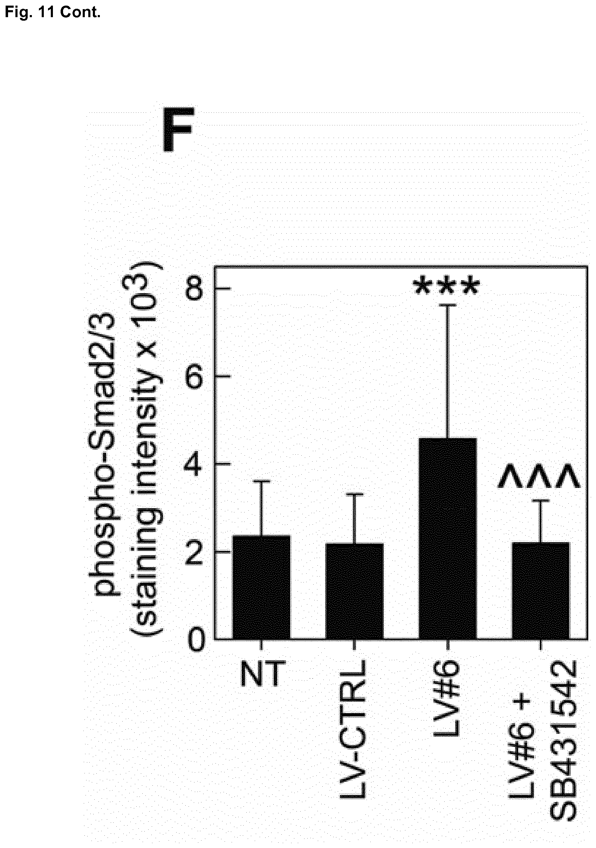

**Figure 11****.** GARP controls TGF-β1 secretion and activation
   (A, B) mASCs, not transduced (NT), control siRNA (control) and specific GARP siRNA (#3 and #6), were cultured for 48 hours and TGF-β1 levels were analyzed in supernatants that are activated (A) or not activated (B). (C, D) NT, LV-CTRL and LV#6 mASCs were cultured for 48 hours in the presence (white-colored bars) or absence (black-colored bars) of SB431542 (10 µM). IL-11 expression (C) and cnn-1 expression (D) were analyzed by means of qPCR. The results are shown as the average (SEM) of 4 independent experiments. *=*p*<0.05 vs. LV-CTRL. ^= p<0.05 vs. LV#6 (E) NT, LV-CTRL and LV#6 mASCs were cultured in 8-well plates (5000 cells/well) in medium supplemented with 0.2% FCS in the presence or absence of SB431542 (10 µM) for 48 hours. The cells were fixed and made permeable with methanol and stained for phospho-Smad2/3 and prepared using the Slowfade® Gold anti-fade reagent (Life Technologies) containing DAPI. Images were captured using a Zeiss LSM 710 confocal microscope. A representative experiment out of two individual experiments is shown. The results are shown as the average (SD). ***=p<0.001 vs. LV-CTRL; ^^^=p<0.001 vs. LV#6.

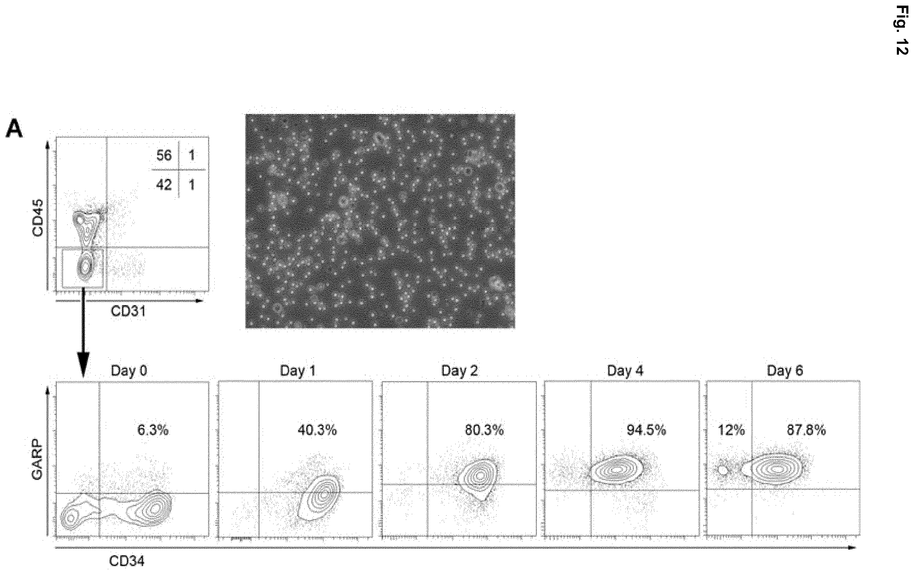

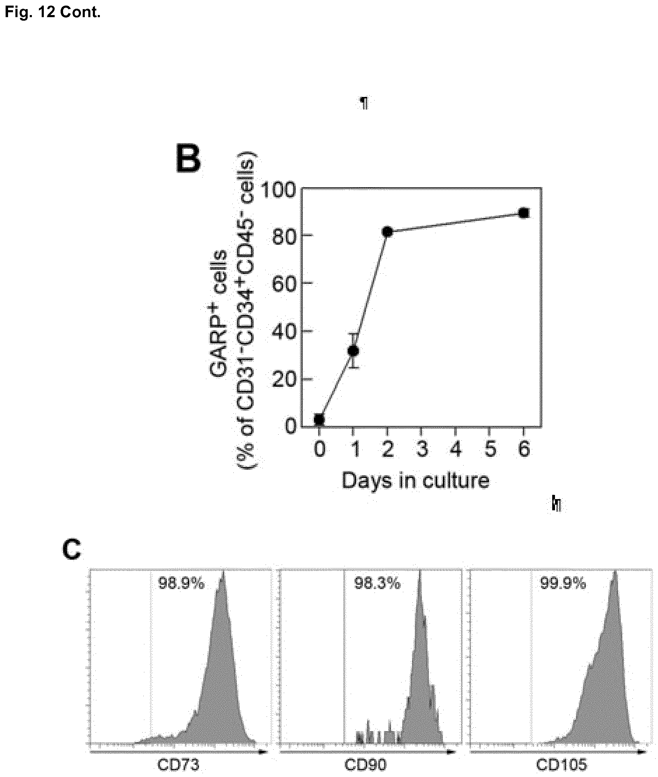

**Figure 12****.** GARP expression is induced in human ASCs *in vitro.*
   (A, B) Adipose tissue samples from healthy individuals were digested with type I collagenase and the cell suspensions were stained with CD45-, CD31-, CD34- and GARP-specific antibodies before (day 0) and 1, 2, 4 and 6 days after seeding in 6-well plates and analyzed by flow cytometry. The results are shown as the average (SEM) of two individual experiments. (C) On day 6, the adhered cells were labeled with CD73-, CD90- and CD105-specific antibodies, and analyzed by means of flow cytometry.

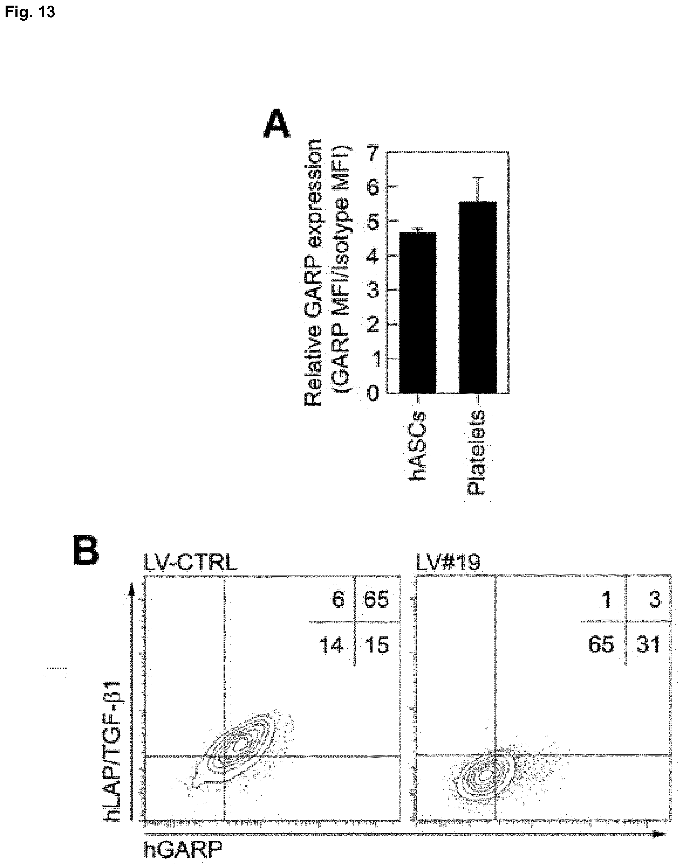

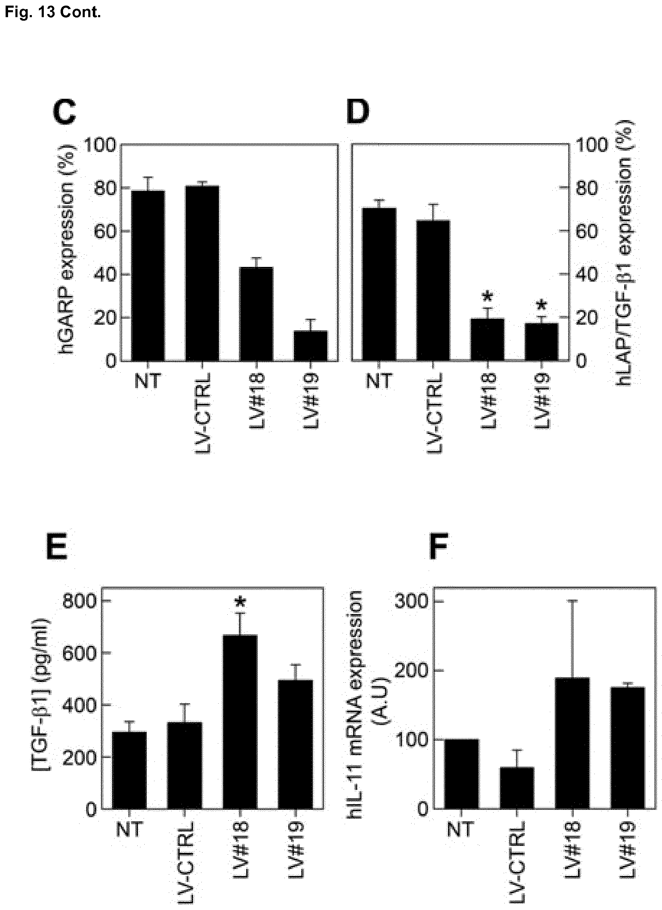

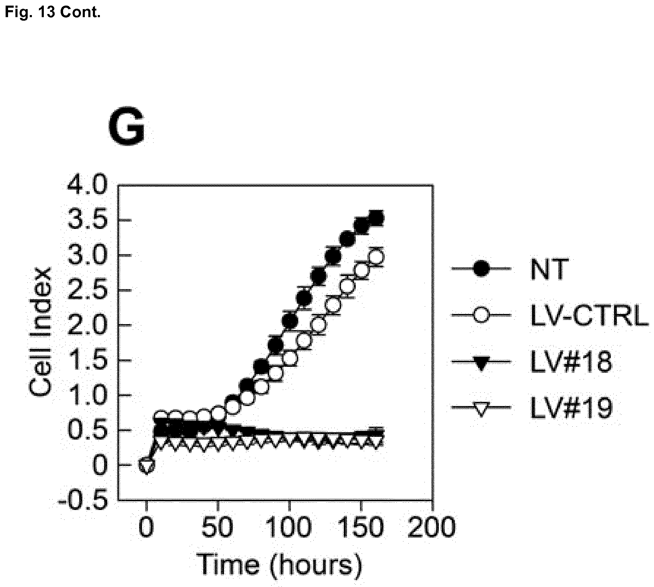

**Figure 13****.** GARP expression in human ASCs
   (A) Human ASCs and platelets were labeled for GARP expression and analyzed by means of flow cytometry. To compare the relative levels of GARP in both cell types, the ratio of GARP MFI/Isotype MFI (top) was calculated. GARP staining (gray-colored histograms) and isotype control (white-colored histograms) of hASCs and platelets (bottom) is shown. (B, C, D) Human ASCs were transduced with lentiviral vectors expressing a non-specific shRNA (LV-CTRL) or specific shRNAs for human GARP (LV#18 and LV#19). (B) Dot plots representative of the co-staining of LAP/TGF-β1/GARP in hASC LV-CTRL (left) and hASCs LV#19 (right). (C, D) The quantification of GARP expression (C) and LAP/TGF-β1 expression (D) in NT hASCs, LV-CTRL, LV#18 and LV#19 is shown as the average (SEM) of 3 independent experiments. *=p<0.05 vsA) or non-activated hASCs (B). (C, D) NT, LV-CTRL and LV#6 mASCs were cultured for 48 hours in the presence (white-colored bars) or absence (black-colored bars) of SB431542 (10 µM). IL-11 expression (C) and cnn-1 expression (D) were analyzed by means of qPCR. The results are shown as the average (SEM) of 4 independent experiments. (E, F) NT, LV-CTRL, LV#18 and LV#19 hASCs were cultured for 72 hours and the TGF-β1 levels were analyzed in activated supernatants by means of ELISA (E) and IL-11 expression was analyzed by qPCR (F). The results are shown as the average (SEM) of 2-4 independent experiments. *=p<0.05 vs. LV-CTRL. (G) Human ASC NT (black-colored circles), LV-CTRL (white-colored circles), LV#18 (black-colored triangles) and LV#19 (white-colored triangles) were added (800 cells/well) to 16-well E-plates and proliferation was monitored in real time using the xCelligence system. The results are shown as the average (SD) of four experimental replicas.

### DETAILED DESCRIPTION OF THE INVENTION

### CELLS

The MSCs can be isolated from various tissues, including among others, bone/bone marrow, fat, Wharton's jelly, umbilical cord blood, placenta and pancreas. The "immunosuppression" property of human MSCs makes them a promising candidate for use thereof in allogeneic cell therapy. In this sense, the use of allogeneic MSCs for tissue repair can become a promising alternative to current tissue grafting methods. Furthermore, an allogeneic approach would allow isolating MSCs from any donor, expanding and cryopreserving same, providing a readily available source of progenitors for use thereof in cell replacement therapy. Furthermore, the immunomodulatory properties of MSCs have allowed the possibility of establishing allogeneic MSC banks for tissue regeneration.

The authors of the present invention have demonstrated that the GARP marker, also referred to as LRRC32, is a surface marker of activated stromal MSCs, particularly in mesenchymal stromal cells derived from adipose tissue (ASC). This protein is an excellent marker for the isolation and/or detection of MSCs from bone marrow. Additionally, the authors of the present invention have demonstrated that the MSCs having GARP surface marker have a pronounced immunomodulatory activity.

Therefore, a first aspect of the invention relates to the *in vitro* use of the GARP protein (glycoprotein-A repetitions predominant protein) for the identification and optionally isolation of activated mesenchymal stem cells.

A preferred embodiment of the first aspect of the invention relates to an *in vitro* method for the identification and/or isolation of a mesenchymal stem cell from a biological sample comprising said mesenchymal stem cells, comprising the detection of GARP on the surface of said cell and the isolation of said MSCs based on the expression of said protein on the surface thereof (LRRC32 or GARP). Preferably, this embodiment is carried out based on the use of a specific reagent capable of detecting *GARP.*

The term "specific reagent" refers to a member of a specific binding pair. Members of a specific binding pair include in addition to binding pairs formed by antigens and antibodies, pairs formed by MHC antigens and T-cell receptors, complementary nucleotide sequences, as well as peptide ligand and receptor pairs. Specific binding pairs include analogues, fragments and derivations of a specific member of the binding pair. In this sense, the use of antibodies as affinity reagents is of particular interest. The production of specific monoclonal antibodies will be obvious for any person having average skill in the art. In experiments for the identification or separation of cell populations, labeling with the antibodies is performed. To that end, labels or markers that are used include, but not limited to, magnetic particles, biotin and fluorochromes which will allow the identification or separation of that cell type to which the antibody has bound. In this manner, for example, the analysis of the cell population by means of flow cytometry allows using in one and the same sample different antibodies labeled with fluorochromes emitting at a different wavelength, such that the specific profile of the population for those surface markers can be known, as well as separation by the set of markers used can be carried out.

The separation of populations having the phenotype of interest can be carried out, for example, but without limitation, by means of affinity separation techniques, including among others: magnetic separation (using magnetic particles coated with specific antibodies), affinity chromatography, cytotoxic agents bound to monoclonal antibodies or used together with monoclonal antibodies, and panning with the antibody associated with a solid support, as well as by means of other suitable techniques. A more precise separation would be obtained by means of flow cytometry, a technique which allows separating cell populations according to staining intensity, together with other parameters such as cell size and cellular complexity.

Flow cytometry is a cell analysis technique which involves measuring the light scattering and fluorescence characteristics of cells as they are passed through a light beam. In flow cytometry analysis, the cells are placed in the presence of a compound that binds specifically to said marker, such as for example, an antibody, and the cells are classified depending on the compound. If the compound is labeled with fluorescence agent, it refers strictly to flow cytofluorometers (those known as "cytometers" or "FACS" (Fluorescence Analyzer Cell Sorter). Flow cytometers can analyze particles according to their fluorescence and size. Those known as sorters can also purify populations according to specific characteristics.

In flow cytometry, for a marker to be considered positive, the specific signal observed must be 10% greater, preferably 20%, 30%, 40%, 50%, 60%, 70%, 80%, 90%, 500%, 1000%, 5000%, and even more preferably 10000% greater than the background signal intensity. Signal intensity can be identified using conventional methods, such as for example, a Beckman Coulter Epics XL FACS system used with commercially available antibodies and standard protocols known in the state of the art. Background signal is defined as the signal intensity given by a non-specific antibody of the same isotype as the specific antibody used for detecting the surface marker in conventional FACS analyses.

Once having put the method of the invention into practice, an isolated mesenchymal stem cell expressing the GARP protein on its surface is obtained as a result.

Also described is an isolated mesenchymal stem cell, hereinafter mesenchymal stem cell, that can be obtained by the method of the first aspect of the invention comprising the detection of GARP (glycoprotein-A repetitions predominant protein) on the surface of said cell and the isolation of said cell based on the expression of said protein.

In the present invention, "activated mesenchymal cells" is understood as those MSCs having increased immunomodulatory, proliferative and migratory activity in relation to non-activated MSCs.

In the present invention, "identification" is understood as the process which allows recognizing an activated mesenchymal stem cell, present in a sample and distinguishing it from the rest of cells present in said sample. Likewise, the term "isolation" of an activated mesenchymal stem cell, refers to the separation of said cell from the rest of the components existing in a sample. It is considered that a mesenchymal stem cell is isolated when the amount of said cells with respect to the rest of the components of the medium is greater than 60%, preferably greater than 70%, 80%, 90%, 95% or 99%.

As it is used herein, the term "mesenchymal stem cell" (MSC) or "stromal stem cells" refers to a mesenchymal stem cell that adheres to plastic and shows self-renewal, proliferation, differentiation (adipocytes, osteocytes and chondrocytes) and immunomodulatory capacity.

The sample from which the MSCs are obtained can come from an individual. As it is used herein, the term "individual" is the same as the term "subject" and includes any animal, particularly, vertebrate animals, preferably mammals, such as horses, pigs, rabbits, cats, sheep, dogs, cows, human beings, etc., in a particular embodiment, the peripheral blood or the blood products thereof is of human origin.

As it is used herein, the term GARP (garpin; glycoprotein A repetitions predominant; leucine-rich repeat-containing protein 32; LRRC32 or D11S833E) is a gene encoding a type I membrane protein containing 20 leucine-rich repeats. It is located in chromosome 11 (11q13.5-q14).

In the context of the present invention, the GARP protein is also defined by a nucleotide or polynucleotide sequence, making up the coding sequence of the protein listed in SEQ ID NO: 1, and comprising various variants from:
a) Nucleic acid molecules encoding a polypeptide comprising the amino acid sequence of SEQ ID NO: 1,
b) Nucleic acid molecules the complementary strand of which hybridizes with the polynucleotide sequence of a),
c) Nucleic acid molecules the sequence of which differs from a) and/or b) due to genetic code degeneration,
d) Nucleic acid molecules encoding a polypeptide comprising the amino acid sequence with an identity of at least 80%, 90%, 95%, 98% or 99% with SEQ ID NO: 1, and in which the polypeptide encoded by said nucleic acids has the activity and structural characteristics of the GARP protein. The nucleotide sequences encoding the GARP protein include, among others, the sequence listed in GenBank (NCBI) NM_001128922.1, in sequence SEQ ID NO: 2.
   SEQ ID NO: 1 
MRPQILLLLALLTLGLAAQHQDKVPCKMVDKKVSCQVLGLLQVPSVLPPDTETLDLSGNQLRSIL
ASPLGFYTALRHLDLSTNEISFLQPGAFQALTHLEHLSLAHNRLAMATALSAGGLGPLPRVTSLD
LSGNSLYSGLLERLLGEAPSLHTLSLAENSLTRLTRHTFRDMPALEQLDLHSNVLMDIEDGAFE
GLPRLTHLNLSRNSLTCISDFSLQQLRVLDLSCNSIEAFQTASQPQAEFQLTWLDLRENKLLHFP
DLAALPRLIYLNLSNNLIRLPTGPPQDSKGIHAPSEGWSALPLSAPSGNASGRPLSQLLNLDLSY
NEIELIPDSFLEHLTSLCFLNLSRNCLRTFEARRLGSLPCLMLLDLSHNALETLELGARALGSLRT
LLLQGNALRDLPPYTFANLASLQRLNLQGNRVSPCGGPDEPGPSGCVAFSGITSLRSLSLVDN
EIELLRAGAFLHTPLTELDLSSNPGLEVATGALGGLEASLEVLALQGNGLMVLQVDLPCFICLKR
LNLAENRLSHLPAWTQAVSLEVLDLRNNSFSLLPGSAMGGLETSLRRLYLQGNPLSCCGNGW
LAAQLHQGRVDVDATQDLICRFSSQEEVSLSHVRPEDCEKGGLKNINLIIILTFILVSAILLTTLAA
CCCVRRQKFNQQYKA
   SEQ ID NO: 2

ggtggggcagctgagcggcctgctcctcctcgggccgtgaccccggggtctggcgcggggtgggacccgggggcgggtttgcgca
aaatgtgccgagactgcccgggagaggagctgcggccgcgctgagccagagccatgagaccccagatcctgctgctcctggccct
gctgaccctaggcctggctgcacaacaccaagacaaagtgccctgtaagatggtggacaagaaggtctcgtgccaggttctgggcc
tgctccaggtcccctcggtgctcccgccagacactgagacccttgatctatctgggaaccagctgcggagtatcctggcctcacccctg
ggcttctacacagcacttcgtcacctggacctgagcaccaatgagatcagcttcctccagccaggagccttccaggccctgacccac
ctggagcacctcagcctggctcacaaccggctggcgatggccactgcgctgagtgctggtggcctgggcccccctgccacgcgtgac
ctccctggacctgtctgggaacagcctgtacagcggcctgctggagcggctgctgggggaggcacccagcctgcataccctctcact
ggcggagaacagtctgactcgcctcacccgccacaccttccgggacatgcctgcgctggagcagcttgacctgcatagcaacgtgct
gatggacatcgaggatggcgccttcgagggtctgccccgcctgacccatctcaacctctccaggaattccctcacctgcatctccgact
tcagcctccagcagctgcgggtgctagacctgagctgcaacagcatcgaggcctttcagacggcctcccagccccaggctgagttcc
agctcacctggcttgacctgcgggagaacaaactgctccatttccccgacctggccgcgctcccgagactcatctacctgaacttgtcc
aacaacctcatccggctccccacagggccaccccaggacagcaagggcatccacgcaccttccgagggctggtcagccctgccc
ctctcagcccccagcgggaatgccagcggccgcccccttcccagctcttgaatctggatttgagctacaatgagattgagctcatccc
cgacagctttcttgagcacctgacctccctgtgcttcctgaacctcagcagaaactgcttgcggacctttgaggcccggcgcttaggctc
cctgccctgcctgatgctccttgacttaagccacaatgccctggagacactggaactgggcgccagagccctggggtctctgcggac
gctgctcctacagggcaatgccctgcgggacctgcccccatacacctttgccaatctggccagcctgcagcggctcaacctgcaggg
gaaccgagtcagcccctgtgggggggccagatgagcctggcccctccggctgtgtggccttctccggcatcacctccctccgcagcct
gagcctggtggataatgagatagagctgctcagggcaggggccttcctccacaccccactgactgagctggacctttcttccaatcctg
ggctggaggtggccacgggggccttgggaggcctggaggcctccttggaggtcctggcactgcagggcaacgggctgatggtcctg
caggtggacctgccctgcttcatctgcctcaagcggctcaatcttgccgagaaccgcctgagccaccttcccgcctggacacaggctg
tgtcactggaggtgctggacctgcgaaacaacagcttcagcctcctgccaggcagtgccatgggtggcctggagaccagcctccgg
cgcctctacctgcaggggaatccactcagctgctgcggcaatggctggctggcagcccagctgcaccagggccgtgtggacgtgga
cgccacccaggacctgatctgccgcttcagctcccaggaggaggtgtccctgagccacgtgcgtcccgaggactgtgagaagggg
ggactgaagaacatcaacctcatcatcatcctcaccttcatactggtctctgccatcctcctcaccacgctggccgcctgctgctgcgtcc
gccggcagaagtttaaccaacagtataaagcctaaagaagccgggagacactctaggtcagtgggggagcctgaggtacagaga
agagtgaggactgactcaaggtcacacagtgatccgggatcccagaactctggtctccaaattacagcccaggacacctttctctgcc
gcctgctgcatcagtgggtgacccccttcccgggctgcactttgggtccagctgtggaagccagaagttgggcggtttcagggacagc
cgagaataatgttgacctgtcagatcaacaaatcttcactgagcatgtattttgtgccacaccctgctctgggcactgggaatgctgga
aatgagatacattcccgccctcaagaatctcccagtctggtaggagagagtgctgcagagccacgtggccgccacgcagtgtgctta
gggcctgaggtgtgaaagcccagggctccagagctcggcaggccccgctggtttggtgcggtgagtcctgccccggctgtgccagg
gtgagggagggccaagcccaggaggatttgtctgagacatttccaagcagactgtttgtcacgtcttctgagaatgactttcagtctctct
gaaaatgaaaagcttaggaccagaagagagaattggagctgtacgagtgtgtctcggatctggtgttgttaggtgggccacggcggc
tccagcagggtctggttaaggggtccagcccagcactggaccattccgtctcctgctctggacttgccctctcccttcctggcactctcat
gttgcataccctgaccccagtgctgctctaagcaccgtccctgcccagccccacttctccatcgcagccccaccttggctgctgagcca
ggagctaaaaccttagatatctggttctgttttgcacccagcttggcagatgtggatttgaatccaagccttgtgtctgcccctatgtgacag
ctctatattttatccccgttttataaaagaggaaactgaagttctgaaaatctccttccagggccccagctaactaatgccataggtgagat
tcaaaccttcatccttctgtctccagggcctgatctttaccactgcaggggctgcaggccgttaagtggacaggaagtggccccacata
gcccgagcagggtctggaagcatcctgtgctgtgcacacctgctctctcctctctcccaggcaggcagctgcaggcgctctcctccttct

ctgcctgtttccctcctcccttcctttccaccctggtgtgggttctcctgttctctctgtgctcttgcattctctcattcccttttcctctattgagcaga
gcctggagtttgagactatggaatccaacctccccattgcacagatggggaaactgaggcttaggaagagaatgaaacttgtggaga
gcttatacagagcctctgggggaaaaaagagcccttatttgtggggtgagattggggggttggaccagagtgatgtcctctctcagctatc
acatcacaagataatgctggctccaaacttcctttctgtgcctcatcatgcaaggatctttttccctcttacaaaaacaggtaaaagcct
cacccagatgacccccatccctcataccatggagtcatgagctgtctgggaagaatggacgtgctgggaccaactcaagaccttgtttt
gctgtcttcatcatcttacctgtgcttggcccacagtctggctcatgatgtgggctcagtaatgtgcgagaaagtgaaaatgccactctctc
caccccattttacagaggagaacaccaaggcccagaggaagttaagggagagtcaatgggcagagccagggctaggccctggt
ggtgtgtggagcacccaggcagacccagtcctggttgggatcacacccacgggtgctactgcacgtaacactcctccttaggcctgg
aggccaaggtgtgggtccccacgcctgatctttgaaaacactacacagggctgctgtcacttcccagggcccaggcctcagcccag
gcctcgggaccaactctttgtataacctacctgaatgtattaaaaactaattttggagaagcaaaaaaaaaaaaaaa

Variant sequences contemplated in the context of the present invention include proteins showing at least 60%, 65%, 70%, 72%, 74%, 76%, 78%, 80%, 90%, or 95% of similarity or identity with SEQ ID NO: 1. The degree of identity between two polypeptides is determined using algorithms run in a computer and methods that are widely known by persons skilled in the art. The identity between two amino acid sequences is preferably determined using the BLASTP algorithm (BLAST Manual, Altschul, S. et al., NCBI NLM NIH Bethesda, Md. 20894, Altschul, S., et al., J., 1990, Mol. Biol. 215:403-410).

Therefore, GARP would act as a surface marker of mesenchymal cells, and preferably, activated mesenchymal cells. "Marker" is understood as a protein distinguishing one cell (or group of cells) from another cell (or group of cells). For example, a protein which is expressed on the surface of precursor cells but not in other cells of a cell population acts as a marker protein for the precursor cells. Marker is usually a cell surface antigen, such that antibodies that bind with the marker protein can be used in cell selection methods, for example, for producing a cell population rich in cells expressing the marker protein. Additionally, the cell of the invention can contain other cell markers characteristic of MSCs. In a particular embodiment, the mesenchymal stem cell of the invention expresses, in addition to GARP, one or more plasma membrane markers selected from the group consisting of CD11b, CD14, CD34, CD45 (negative) and Sca-1 (mice), CD29, CD44, CD73, CD90, CD105 (mice and humans).

The detection of the marker and the subsequent isolation of the cells expressing said marker, i.e., the isolation of CMMs, can be performed by means of any method known in the state of the art.

In the identification or isolation assays, the cell population is contacted with a specific reagent that is labeled or not labeled depending on if the assay is performed by means of a direct or indirect detection method, respectively.

Any of the steps and methods for isolating the cells can be performed manually, if desired. Another option is that the process of isolating the cells can be facilitated through a suitable device.

Likewise, the cells can come from any animal having blood circulation, preferably, mammals, more preferably, non-human primates, even more preferably, humans, and they can be autologous, allogeneic or xenogeneic cells depending on the individual to whom they will be administered. In a particular embodiment, the mesenchymal stem cell is of human origin.

Also described is an isolated cell population, hereinafter isolated cell population, comprising at least one mesenchymal stem cell. Preferably, the isolated cell population is a substantially pure cell population comprising at least 50%, 60%, 70%, 80%, 85%, 90%, 91%, 92%, 93%, 94%, 95%, 96%, 97%, 98% or 99% of MSC type cells isolated by a method comprising the detection of GARP or obtained from one or more MSC cells isolated by a method comprising the detection of GARP. The cells are capable of being expanded *ex vivo*, i.e., after being isolated, said cells can be maintained in a culture medium and left to proliferate. Said medium consists of, for example, Dulbecco's Modified Eagle Medium (DMEM), antibiotics and glutamine, and is supplemented with fetal bovine serum (FBS). It depends on the expertise of the person skilled in the art to modify or modulate the concentrations of the media and/or the supplements for the media as required for the cells used. The sera often contain cell components and factors which are required for viability and expansion. Among others, the examples of sera can include FBS, bovine serum (BS), calf serum (CS), fetal calf serum (FCS), newborn calf serum (NCS), goat serum (GS), horse serum (HS), porcine serum, sheep serum, rabbit serum, rat serum (RS), etc. If the cells are of human origin, supplementing the cell culture medium means with human serum, preferably of autologous origin, is also contemplated. It is understood that the sera can be inactivated by heat at 55-65°C if deemed necessary for inactivating the components of the complement cascade. Modulation of serum concentrations, removal of serum from the culture medium to promote the survival of one or more desired cell types, can also be used. The cells can be expanded in a culture medium having a defined composition, in which the serum is replaced with a combination of serum albumin, serum transferrin, selenium and recombinant proteins including, although without limitation, insulin, platelet-derived growth factor and basic fibroblast growth factor (bFGF).

Many cell culture media already contain amino acids; nevertheless, some required being supplemented before culturing the cells. Said amino acids include, although without limitation, L-alanine, L-arginine, L-aspartic acid, L-cysteine, L-cystine, L-glutamic acid, L-glutamine, L-glycine and other similar amino acids. Antimicrobial agents are also normally used in cell culture to mitigate bacterial, mycoplasma and fungal contamination. Antibiotic or antimycotic compounds used are normally mixtures of penicillin and streptomycin, which can also include, although without limitation, amphotericin, ampicillin, gentamicin, bleomycin, hygromycin, kanamycin, mitomycin, etc. Hormones can also advantageously be used in the cell culture and include, although without limitation, D-aldosterone, diethylstilbestrol (DES), dexamethasone, b-estradiol, hydrocortisone, insulin, prolactin, progesterone, somatostatin/human growth hormone (HGH), etc.

The maintenance conditions of the cells can also contain cellular factors allowing the cells to remain in an undifferentiated form. It is obvious for a person skilled in the art that before differentiation, supplements inhibiting cell differentiation must be removed from the culture media. It is also obvious that not all cells will require these factors. In fact, these factors can cause unwanted effects, depending on the cell type.

If desired, the cells can be clonally expanded using a method suitable for cloning cell populations. For example, a proliferated cell population can be physically collected and seeded in a separate plate (or the wells of multi-well plate). Another option is that the cells can be subcloned in a multi-well plate in a statistical ratio to facilitate the operation of placing a single cell in each well (for example, from about 0.1 to about one cell/ well or even about 0.25 to 0.5 cells/well, such as for example, 0.5 cells/ well). The cells can of course be cloned at a low density (for example, in a Petri dish or another suitable substrate) and they can be isolated from cells using devices such as cloning rings. The production of a clonal population can be expanded in any suitable culture medium. In any case, the isolated cells can be cultured to a suitable point when their development phenotype can be evaluated.

### INDUCING AGENTS, POLYNUCLEOTIDES AND GENE CONSTRUCTS

A third aspect of the invention relates to a GARP expression inducing agent, selected from the list consisting of:
a) an organic molecule;
b) a DNA or RNA molecule;
c) an antisense oligonucleotide,
d) an antibody; and/or
e) a ribozyme;
or any combination thereof, for increasing the immunomodulatory effect of the MSCs.

The GARP expression inducing agent can be, preferably, a DNA or RNA molecule or polynucleotide which, when introduced in an MSC, increases the immunomodulatory effect of said MSC, by increasing the GARP expression on the surface thereof. Said DNA or RNA polynucleotide will have a nucleotide sequence with an identity of at least 50%, more preferably 60%, 70%, 80%, 90%, 95%, 98% and even much more preferably 99% with the sequence SEQ ID NO: 2.

Therefore, a fourth aspect of the invention relates to an isolated RNA or DNA polynucleotide, hereinafter polynucleotide of the invention, capable of being translated into an amino acid sequence comprising a peptide having an identity of at least 60%, more preferably 80%, 85%, 90%, and even more preferably 95% with the amino acid sequence of the GARP protein (SEQ ID NO: 1), for increasing the immunomodulatory effect of the MSCs as well as the expression level of the GARP protein. More preferably, it refers to an isolated RNA or DNA polynucleotide capable of being translated into an amino acid sequence comprising a peptide having an identity of at least 99% with the amino acid sequence of the GARP protein (SEQ ID NO: 1).

A fifth aspect of the invention relates to a DNA or RNA gene construct, hereinafter gene construct of the invention, comprising at least one of the following types of sequences:
a) nucleotide sequence comprising at least one polynucleotide of the invention for *in vitro* or *in vivo* transcription; or
b) nucleotide sequence corresponding to a gene expression vector or system comprising a polynucleotide of the invention operatively linked with at least one promoter directing the transcription of said nucleotide sequence, and with other sequences necessary or suitable for the transcription and the suitable regulation thereof in time and place.

Preferably, the gene construct is a vector, hereinafter vector of the invention, more preferably said vector is a lentiviral vector.

A large number of these constructs, expression vectors or systems can be produced by conventional methods known by the persons skilled in the art and form part of the present invention.

A "vector" is a replicon or an integrative vector to which another polynucleotide segment has been bound to carry out the replication and/or expression of the bound segment.

A "replicon" is any genetic element that behaves like an autonomous polynucleotide replication unit within a cell; i.e., it is capable of replicating under its own control.

An integrative vector is any genetic element which integrates itself in the cell genome and remains stable therein.

A "lentiviral vector" is an integrative vector derived from lentivirus (non-oncogenic retrovirus capable of infecting all type of cells, both quiescent cells and dividing cells). Lentiviral vectors can insert DNA fragments of intermediate size which can reach up to 8000 bp. The main drawback of these vectors is that their integration into the host cell genome is completely random. Today, most of these vectors are derived from human immunodeficiency virus (HIV).

"Control sequence" refers to polynucleotide sequences which are necessary to carry out the expression of the sequences to which they are ligated. The nature of said control sequences differs depending on the host organism; in prokaryotes, said control sequences generally include a promoter, a ribosomal binding site, and termination signals; in eukaryotes, said control sequences generally include promoters, termination signals, enhancers and, sometimes, silencers. It is envisaged that the term "control sequences" includes at least all the components the presence of which is necessary for the expression, and it can also include additional components the presence of which is advantageous.

As used herein, the term "promoter" refers to a DNA region located upstream of the transcription start point. There are promoters which initiate transcription in a specific type of tissues and they are referred to as "tissue-specific" promoters. An "inducible" or "repressible" promoter is a promoter that is under the control of the surrounding. Tissue-specific promoters, cell type-specific promoters, or inducible promoters are a type of promoters referred to as "non-constitutive" promoters. A "constitutive" promoter is a promoter which is active under most conditions of the surrounding.

"Operatively bound" refers to a juxtaposition in which the components thus described have a relationship that allows them to function in the intended manner.

A control sequence "operatively bound" to a sequence which is transcribed into the nucleotide sequence of the invention is ligated such that the expression of the coding sequence is achieved in conditions compatible with the control sequences.

A "coding sequence" is a polynucleotide sequence which is transcribed into an mRNA and/or translated into a polypeptide when it is under the control of suitable regulatory sequences. The limits of the coding sequence are determined by means of a translation start codon at the 5' end and a translation termination codon at the 3' end. A coding sequence can include, but is not limited to, mRNA, cDNA, and recombinant polynucleotide sequences.

The terms "polynucleotide" and "nucleic acid" are used interchangeably to refer to polymeric forms of nucleotides of any length, both ribonucleotides (RNA) and deoxyribonucleotides (DNA). The terms "amino acid sequence", "peptide", "oligopeptide", "polypeptide" and "protein" are used interchangeably to refer to a polymeric form of amino acids of any length, which may or may not be chemically or biochemically modified.

A sixth aspect of the invention relates to an isolated cell, hereinafter second isolated cell of the invention, comprising the isolated RNA or DNA polynucleotide, or the gene construct of the invention. Preferably, the isolated cell is a mesenchymal stem cell of a mammal, and more preferably it is a human mesenchymal stem cell.

### COMPOSITION

In addition, the cells or the cell population can form part of a pharmaceutical composition.

Therefore, a composition is described, the composition comprising the mesenchymal stem, the cell population or the substantially pure cell population, the isolated RNA or DNA polynucleotide, the gene construct or the second isolated cell, all as described above. Preferably, the composition is a pharmaceutical composition optionally including more than one active ingredient as well as pharmaceutically acceptable vehicles or excipients.

The term "pharmaceutically acceptable vehicle" refers to a vehicle that must be approved by a regulatory agency of the federal government or state government or listed in the United States Pharmacopeia or the European Pharmacopeia, or another pharmacopeia generally recognized for use in animals, and more specifically in humans.

The term "vehicle" refers to a diluent, adjuvant, excipient or carrier which must be used to administer the cells or the cell population or said composition comprising stem cells which can be obtained according to the method of the invention; obviously, said vehicle must be compatible with said cells. Non-limiting, illustrative examples of said vehicle include any physiologically compatible vehicle, for example, isotonic solutions (e.g., 0.9% NaCl sterile saline solution, phosphate buffered saline (PBS) solution, Ringer-lactate solution, etc.), optionally supplemented with serum, preferably with autologous serum; cell culture media (e.g., DMEM, etc.); or, alternatively, a solid, semisolid, gelatinous or viscous support medium, such as collagen, collagen-glycosamino-glycan, fibrin, polyvinyl chloride, polyamino acids, such as polylysine, or polyornithine, hydrogels, agarose, silicone or dextran sulfate. Likewise, if desired, the support medium can contain, in specific embodiments, growth factors or other agents. If the support is solid, semisolid or gelatinous, the cells can be introduced in a liquid phase of the vehicle which is subsequently treated such that it becomes a more solid phase.

The pharmaceutical composition, if desired, can also contain, when necessary, additives for increasing, controlling or otherwise directing the desired therapeutic effect of the cells, which comprise said pharmaceutical composition, and/or auxiliary substances or pharmaceutically acceptable substances, such as buffering agents, surfactants, cosolvents, preservatives, etc. To stabilize cell suspension, it is also possible to add metal chelating agents. The stability of the cells in the liquid medium of the pharmaceutical composition can be improved by means of adding additional substances, such as for example, aspartic acid, glutamic acid, etc. Said pharmaceutically acceptable substances which can be used in the pharmaceutical composition are generally known by the persons skilled in the art and are normally used in the production of cell compositions. Examples of suitable pharmaceutical vehicles are described, for example, in "Remington's Pharmaceutical Sciences", by E.W. Martin. Additional information about said vehicles can be found in any manual of pharmaceutical technology (Galenic Pharmacy).

The pharmaceutical composition will contain a prophylactically or therapeutically effective amount of the cells or of the cell population, preferably, a substantially pure cell population, to provide the desired therapeutic effect. As it is used herein, the term "therapeutically or prophylactically effective amount" refers to the amount of cells contained in the pharmaceutical composition which is capable of producing the desired therapeutic effect and, it will generally be determined, among others factors, by the actual characteristics of the cells and the desired therapeutic effect sought. In general, the therapeutically effective amount of the cells which must be administered will depend, among other factors, on the actual characteristics of the subject, the severity of the disease, the administration route, etc. For this reason, the doses mentioned must only be considered as a guideline for the person skilled in the art who must adjust this dose depending on the factors described above. As a non-limiting, illustrative example, the pharmaceutical composition can be administered as a single dose containing about between 1x10⁵ and 10x10⁶ of the cells per kilo of body weight of the recipient, and more preferably between 5x10⁵ and 5x10⁶ of the cells per kilo of the body weight of the recipient, in an even more preferred embodiment, said pharmaceutical composition will contain about between 1x10⁶ and 2x10⁶ of the cells per kilo of the body weight of the recipient, depending on the factors described above. The dose of cells can be repeated depending on the state of progress of the patient, in time intervals of days, weeks or months which must be established by the specialist in each case.

The pharmaceutical composition will be formulated according to the chosen dosage form. The pharmaceutical composition can be prepared in a liquid or gel dosage form, for example, in the form of a suspension, for being injected or infused into the individual. Non-limiting, illustrative examples include the formulation of the pharmaceutical composition in a sterile suspension with a pharmaceutically acceptable excipient, such as a isotonic solution, for example, phosphate buffered saline (PBS) solution, or any other suitable pharmaceutically acceptable vehicle, for the parenteral administration to a subject, e.g., a human being, preferably intravenously, intraperitoneally, subcutaneously, etc., although other alternative administration routes also are possible.

The pharmaceutical composition will be administered to the individual using conventional means. For example, said pharmaceutical composition can be administered to said individual intravenously using suitable devices, such as syringes, catheters (a standard peripheral intravenous catheter, a central venous catheter or a pulmonary artery catheter, etc.), needles, cannulas, etc. The flow of the cells can be controlled by serial inflation and deflation of distal and proximal balloons located within the patient's vasculature, thereby creating temporary flow-free zones which promote cellular therapeutic action. In all cases, the pharmaceutical composition will be administered using equipment, apparatus and devices suitable for the administration of cell compositions and known by the person skilled in the art. As understood by the person skilled in the art, the direct administration of the pharmaceutical composition to the site intended for benefiting from said administration can sometimes be advantageous. Therefore, the direct administration of the pharmaceutical composition to the desired organ or tissue can be achieved by direct administration (e.g., by injection, etc.) in the outer surface of the affected organ or tissue by means of inserting a suitable device, e.g., a suitable cannula, by arterial or venous infusion (including retrograde flow mechanisms) or by other means mentioned in this description or known in the art.

The pharmaceutical composition, if desired, can be stored until the time of use by means of the conventional methods known by the persons skilled in the art. This pharmaceutical composition can also be stored together with additional medicinal products useful in the treatment of diseases, in an active form comprising a combined therapy. For storage within a short time period (less than 6 hours), the pharmaceutical composition can be stored at or below room temperature in a sealed receptacle, complementing or not complementing it with a nutrient solution. Storage within an intermediate time period (less than 48 hours) is preferably performed at 2-8°C, and the pharmaceutical composition will include an iso-osmotic, buffered solution in a container formed by or covered with a material that prevent cell adhesion. Storage within a longer time period is preferably carried out by means of suitable cryopreservation and storage in conditions promoting cellular function retention.

The pharmaceutical composition can be used in a combined therapy useful for the prevention and/or treatment of the disease to be treated. Said additional compounds can form part of the same pharmaceutical composition or can be alternatively supplied in the form of a different composition for simultaneous or successive administration (sequential in the time) with respect to the administration of the pharmaceutical composition.

If desired, the mesenchymal stem cell can be genetically modified by any conventional method including, in a non-limiting, illustrative manner, transgenesis processes, genome eliminations or insertions which modify the expression of genes that are important for the basic properties thereof (proliferation, migration, transdifferentiation, etc.). In this sense it is known, for example, that adult stem cells expanded *ex vivo* or transplanted in damaged tissues age quickly due to the shortening of their telomeres. To prevent this and other phenomena, it may be desirable to transduce the cells using, for example, retroviral particles containing gene constructs the expression of which counteracts the effect of this and other unwanted alterations.

In a preferred embodiment, the mesenchymal stem cell is modified so that it overexpresses the GARP protein on the surface thereof. Therefore, also described is a cell, hereinafter second cell, comprising the isolated polynucleotide as described above, or the gene construct as described above. Preferably, the second cell is a mesenchymal cell, and more preferably, an activated mesenchymal cell.

### USES OF THE MESENCHYMAL STEM CELL, THE CELL POPULATION, THE SUBSTANTIALLY PURE CELL POPULATION, THE ISOLATED RNA OR DNA POLYNUCLEOTIDE, THE GENE CONSTRUCT, THE SECOND ISOLATED CELL AND/OR THE COMPOSITION

The use of MSCs in the treatment of diseases is being studied based on the their *in vitro* antiproliferative properties, their effectiveness in animal models and some isolated results achieved in acute cases of graft-versus-host disease in humans. Phase I/II clinical trials are being developed for studying the use thereof in Crohn's disease and in multiple sclerosis and trials for systemic lupus erythematosus, systemic sclerosis, systemic vasculitis, type 1 diabetes and other autoimmune diseases are planned to be started. The state of the art also discloses the MSC capacity to interact with cells of the immune system for controlling an immune response which, in the case of autoimmune diseases, is responsible for the destruction of different specific tissues or cells causing their deterioration. In these cases, the use of MSCs allows "shutting down" lymphocytes T, B and NK, obtaining an asymptomatic state free of immunosuppressant medicinal products which cause irreversible damage in vital organs such as the kidney in the long term.

In turn, the isolated polynucleotides as described above or the gene constructs as described above can be transduced or transfected into the MSCs, and can therefore be used for overexpressing GARP in the MSCs, improving their immunomodulatory activity and therefore their therapeutic activity.

Therefore, also described is the use of the mesenchymal stem cell, the cell population or the substantially pure cell population, the isolated RNA or DNA polynucleotide, the gene construct or the second isolated cell, all as described above, in the production of a medicinal product. Alternatively, described is the mesenchymal stem cell, the cell population or the substantially pure cell population, the isolated RNA or DNA polynucleotide, the gene construct or the second isolated cell, all as described above, for use in therapy.

Also described is the use of the isolated mesenchymal stem cell, the cell population or the substantially pure cell population, the isolated RNA or DNA polynucleotide, the gene construct or the second isolated cell, all as described above, in the production of a medicinal product for tissue and organ repair or regeneration. Alternatively, it is described the mesenchymal stem, the cell population or the substantially pure cell population, the isolated RNA or DNA polynucleotide, the gene construct or the second isolated cell, all as described above, for use in tissue and organ repair or regeneration.

Also described is the use of the isolated mesenchymal stem cell, the cell population or the substantially pure cell population, the isolated RNA or DNA polynucleotide, the gene construct or the second isolated cell, all as described above, in the production of a medicinal product for immunomodulation. Alternatively, it is described the mesenchymal stem cell, the cell population or the substantially pure cell population, the isolated RNA or DNA polynucleotide, the gene construct or the second isolated cell, all as described above, for use as an immunomodulator. Also described is the use of the MSCs or a cell population in the preparation of a medicinal product for treating an inflammatory disease.

Inflammatory diseases and autoimmune diseases include, without limitation, Addison's disease, alopecia areata, ankylosing spondylitis, hemolytic anemia, pernicious anemia, aphthae, aphthous stomatitis, arthritis, arteriosclerosis, osteoarthritis, rheumatoid arthritis, aspermiogenesis, bronchial asthma, autoimmune asthma, autoimmune hemolysis, Behçet's disease, Boeck's disease, inflammatory bowel disease, Burkitt's lymphoma, Crohn's disease, choroiditis, ulcerative colitis, Celiac disease, cryoglobulinemia, dermatitis herpetiformis, dermatomyositis, insulin-dependent diabetes, juvenile diabetes, autoimmune demyelinating diseases, Dupuytren's contracture, encephalomyelitis, allergic encephalomyelitis, endophthalmia, enteritis allergica, autoimmune enteropathy syndrome, erythema nodosum leprosum, idiopathic facial paralysis, chronic fatigue syndrome, rheumatic fever, glomerulonephritis, Goodpasture's syndrome, Graves' disease, Harnman-Rich's disease, Hashimoto's disease, sudden hearing loss, chronic hepatitis, Hodgkin's disease, paroxysmal hemoglobinuria, hypogonadism, regional ileitis, iritis, leucopenia, disseminated lupus erythematosus, systemic lupus erythematosus, cutaneous lupus erythematosus, lymphogranuloma, infectious mononucleosis, myasthenia gravis, transverse myelitis, primary idiopathic myxedema, nephrosis, sympathetic ophthalmia, orchitis granulomatosa, pancreatitis, pemphigus vulgaris, polyarteritis nodosa, chronic polyarthritis, polymyositis, acute polyradiculitis, psoreasis, purpura, pyoderma gangrenosum, Reiter's syndrome, sarcoidosis, ataxic sclerosis, progressive systemic sclerosis, scleritis, scleroderma, multiple sclerosis, disseminated sclerosis, infertility due to anti-spermatozoa antibodies, thrombocytopenia, thymoma, acute anterior uveitis, vitiligo, AIDS, SCID and Epstein Barr virus associated diseases such as Sjögren's syndrome, AIDS or Epstein Barr virus associated B cell lymphoma, parasitic diseases such as leishmaniasis and immunosuppressed states such as viral infections following transplants, AIDS, cancer, chronic active hepatitis, transplant rejection as a result of tissue or organ transplant and graft-versus-host disease which can result from bone marrow or stem cell transplant.

The immunomodulatory capacity of MSCs is not only important in the treatment of autoimmune diseases, but they are also defined as an indispensable treatment element to promote tolerance towards solid organs such as the heart, lung and kidney. For example, the co-infusion of MSCs during transplant is possible.

Therefore, also described is the use of a mesenchymal stem cell or a cell population for the preparation of a medicinal product for inducing transplant tolerance.

Once the MSCs enter the blood stream, they are capable of detecting molecules secreted by damaged or dying tissues in what is known as "homing" phenomenon. Once in the proximity of the damaged tissue, the cell adheres to the surface of the organ through molecular receptors expressed on the surface of the cell membrane, this starts a surprising series of events allowing the stem cell to be integrated in the damaged organ and to start secreting growth factors locally stimulating the stem cells residing in the affected organ itself, in addition to changing the inflammatory biochemical environment to give rise to a microenvironment that will allow cell regeneration, in which a cell fusion or differentiation process starts, thus being converted into in a physiologically mature cell, in addition to promoting the formation of new blood vessels.

Therefore, also described is the use of a mesenchymal stem cell or a cell population in the preparation of a medicinal product for tissue repair and regeneration.

As it is used herein, the term "active ingredient", "active substance", "pharmaceutically active substance", or "pharmaceutically active ingredient" means any component which potentially provides a pharmacological activity or another different effect in diagnosing, curing, mitigating, treating or preventing a disease, or which affects the structure or function of the human body or the body of other animals. The term includes those components promoting a change in the production of a drug and present therein in an envisaged modified form providing the specific activity or the effect.

As it is used herein, the term "medicinal product" refers to any substance used for the prevention, diagnosis, relief, treatment or cure of diseases in human beings and animals. Another aspect of the invention relates to the use of GARP as a mesenchymal stem cell marker. Throughout the description and the claims the word "comprises" and variants thereof do not intend to exclude other technical features, supplements, components or steps. The following examples and drawings are provided by way of illustration and they are not meant to limit the present invention. Furthermore, the present invention covers all the possible combinations of particular and preferred embodiments herein indicated.

### EXAMPLES OF THE INVENTION

### MATERIALS AND METHODS

### Animals

Balb/c male mice (6-10 weeks, Charles River, Barcelona, Spain) were used to start the ASC or BM-MSC cultures for the generation of splenocyte cell suspensions. All the experiments were performed according to the institutional directives for the care and use of laboratory animals in research and with the approval of the local ethics committee in Hospital Virgen Macarena of Seville, Spain.

### Isolation of mesenchymal stem cells from adipose tissue (mASCs)

To carry out the present experiment, abdominal fat (epididymal) and subcutaneous fat (inguinal) from 6 male Balb/c and C57BL/6 mice (6-10 weeks of age, Charles River, Barcelona, Spain) (Cobo et al., 2013. Cell Transplant 22, 839-854) as well as human subcutaneous fat obtained from healthy individuals were used. Briefly, the fat tissue was aseptically removed, cut into small pieces, resuspended in 2.5 ml of Hank's balanced salt solution (HBSS, Invitrogen, Carlsbad, CA), containing 2 mg/ml of type I collagenase (Sigma Aldrich, St. Louis, MO) per gram of fat tissue and incubated for 30 minutes at 37°C. After washing, the cells were resuspended in complete MesenCult medium (*Stem Cell*, Grenoble, France) containing 20% mouse mesenchymal stem cell stimulating supplements (*Stem Cell*) and 100 U/ml of penicillin/streptomycin (Invitrogen) and seeded at a density of 15,000-30,000 cells/cm² and cultured at an atmosphere of 5% O₂/5% CO₂. Non-adhered cells were removed after culturing for 24 hours. Subsequent passes were seeded at 10,000 cells/cm² in complete MesenCult medium. BM-MSCs were derived by means of the washing of femurs and tibias of BALB/c mice and adding 1.5x10⁶ cells/cm² in cell culture flasks. BM-MSCs were used in step >5. Human ASCs were obtained from Inbiomed (San Sebastian, Spain) and cultured in DMEM supplemented with 10% FCS (Gibco) and 100 U/ml of penicillin/streptomycin (Gibco, Life Technologies). All the experiments with samples obtained from humans were conducted according to the institutional directives and approved by the ethics committee of the HU Virgen de la Macarena.

### Vector production and assessment

The MISSION® shRNA plasmid specific for mouse GARP (LRRC32) (RefSeq: SHCLND-NM_001113379; two different shRNA plasmids specific for mouse LRRC32/GARP, referred to as LV#3 and LV#6, were used), for human GARP (RefSeq: SHCLND-NM_005512; two different shRNA plasmids specific for human LRRC32/GARP, referred to throughout the text as LV#18 and LV#19, were used) and the pLKO.1-puro MISSION® shRNA control plasmid for non-mammals (Sigma) were amplified in One Shot® Stbl3 *E. coli* (Invitrogen) at 30°C. Lentiviral vectors were produced by means of co-transfection of 293T kidney cells with three plasmids: 1. plasmid of the vector, 2. packaging plasmid pCMVΔR8.91, and 3. envelope plasmid pMD.G, using Lipofectamine 2000 (Invitrogen) as described in other research papers (Toscano et al., 2004. Gene Ther. 11, 956-961). The viral supernatants were collected and filtered through a 0.45 µm filter and immediately frozen at -80°C or used for transducing MSCs. The vector particles were concentrated by ultrafiltration at 2000 x g and 4°C, using 100 kDa of centrifuge filter devices as described previously (Frecha et al., 2008. Gene Ther. 15, 930-941) (Amicon Ultra-15, Millipore, Billerica, MA). The assessment of the vector was conducted in 293T cells. The CEWP assessment vector was determined using the percentage of positive eGFP cells by FACS analysis 7 days after transduction. For the assessment of LentiVIP vectors, the transduced cells were lysed and the DNA was extracted after 7-10 days. The number of copies of the vector per cell was determined using Q-PCR. Q-PCR was performed with the MX3005Pro sequence detection system (Stratagene, Santa Clara, CA). For the transfection of mASCs, mASCs from passes 2-3 were harvested and 0.7 x 10⁶ cells were mixed with the concentrated virus and seeded in a well in a 6-well plate. After 5 hours, the viruses were eliminated and the ASCs were collected and again seeded in T75 culture flasks. GARP expression was assayed by flow cytometry 3-4 days after transfection. For the different transduced LV cells, the following primers were used: FW puromycin: 5'- 3'-TGCAAGAACTCTTCCTCACG, RV puromycin: 5'-3' AGGCCTTCCATCTGTTGCT. To determine the standard curve in each experiment, 10-fold increasing amounts of plasmid DNA were used (10² to 1 x 10⁷ copies).

### FACS (GARP AND mbTGFβ; ASC phenotype)

For LAP/TGF-β1 staining, the ASCs were seeded at 5000 cells/cm² and after 24-48 hours, the cells were collected with PBS with 2 mM EDTA. The ASCs were stained with purified anti-mouse LAP/TGFβ1 or anti-human LAP/ TGFβ1 (Biolegend, San Diego, CA), followed by goat anti-APC IgG. For GARP expression, the ASCs were captured by means of TrypLE and stained for murine GARP (Garp-PE) or human GARP (GARP-cFluor660) both from eBioscience. Isotype controls suitable for determining background staining were used. For human platelet GARP staining, blood from healthy volunteers was collected in tubes with EDTA and centrifuged at 400 x g for 7 minutes to obtain the supernatant containing platelets. The platelets were then precipitated at 800 x g for 7 minutes and washed with PBS, again centrifuged at 400 x g to dismiss cell contaminants and counted. 106 human platelets were stained for human GARP (GARP-eFluor660; G14D9) and CD41a -PE (HIP8; eBioscience).

For hASC (human adipose-derived stem cell) characterization, the cells were stained with CD45-FITC, CD31-PE, CD34-PE-Cy7, CD73-APC, CD90-APC, CD105-APC (all from eBioscience).

### qPCR

ASC RNA was obtained using the Trizol reagent (Invitrogen) according to the manufacturer's instructions. The RNA samples were subjected to reverse transcription using first-strand SuperScript (Invitrogen) and Q-PCR was performed using SYBRGreen QuantiTect PCR kit (Qiagen, Valencia, CA) in a Stratagene Mx3005P system. The primers are:
Murine GARP FW 5'-ACCAGATCCTGCTACTCCTG-3'; (SEQ ID NO: 3)
Murine GARP RV:5'-ACGAAGCGCTGTATAGAAGC-3'; (SEQ ID NO: 4)
Human GARP FW:5'-ACAACACCAAGACAAAGTGC-3'; (SEQ ID NO: 5)
Human GARP RV:5'-ACGAAGTGCTGTGTAGAAGC-3'; (SEQ ID NO: 6)
TGF-β1 FW: 5'-TGCGCTTGCAGAGATTAAAA-3'; (SEQ ID NO: 7)
TGF-β1 RV: 5'-AGCCCTGTATTCCGTCTCCT-3'; (SEQ ID NO: 8)
Puromycin FW: 5'-TGCAAGAACTCTTCCTCACG-3'; (SEQ ID NO: 9)
Puromycin RV: 5'-AGGCCTTCCATCTGTTGCT-3'; (SEQ ID NO: 10)
LTBP1 FW: 5'-AACCAGGGTTACAGAGCATC-3'; (SEQ ID NO: 11)
LTBP1 RV: 5'-AGCCCTTATTGTCGTTCAGC-3'; (SEQ ID NO: 12)
IL-11 FW: 5'-TCCTTCCCTAAAGACTCTGG-3'; (SEQ ID NO: 13)
IL-11 RV: 5'-TTCAGTCCCGAGTCACAGTC-3'; (SEQ ID NO: 14)
Murine β-actin FW: 5'-AATCGTGCGTGACATCAAAG-3'; (SEQ ID NO: 15)
β-actin of RV: 5'-ATGCCACAGGATTCCATACC-3'. (SEQ ID NO: 16)
GAPDH FW: 5'-ATGGGGAAGGTGAAGGTCG-3'; (SEQ ID NO: 17)
GAPDH RV: 5'-GGGGTCATTGATGGCAACAATA-3'; (SEQ ID NO: 18)
IL-11 FW: 5'-GACCTACTGTCCTACCTGCG-3' (SEQ ID NO: 19),
IL-11 RV: 5'-AGTCTTCAGCAGCAGCAGTC-3' (SEQ ID NO: 20)

### Proliferation: Celltiter Blue & Celligence

To study ASC proliferation, Roche xCELLigence real time cell analysis system was used (Roche Applied System, Penzberg, Germany). In summary, 100 µl of complete MesenCult medium were added to the wells of untreated 16-well plates (Roche) to obtain background readings, followed by the addition of 100 µl of cell suspension containing 800 cells/well of NT, CTRL, #3 and #6 ASC. Then, the E-plates were placed in the device station in the incubator (normoxic condition; 5% CO₂) for continuously recording impedance, as reflected by the cell index. Proliferation was monitored for 7 days with a medium change on day 4. In some experiments, a TGF-β 1/2/3 neutralizing antibody (R&D Systems, Minneapolis, MN) was added to the wells at 10 µg/ml. Cell proliferation was also assayed using the Celltiter-blue reagent (Promega, Madison, WI). ASCs NT, CTRL, #3 and #6 were seeded in 96-well plates (800 cells/well in 100 µl of complete MesenCult) and cultured at 5% O₂. On days 1, 3 and 7, 20 µl of Celltiter-blue were added to the cells and the plates were incubated for another 4 hours at 37°C, after which 100 µl of the culture supernatants were transferred to an opaque 96-well plate (Greiner, Frickenhausen, Germany) and fluorescence was measured at 560 nm.

### Evaluation of ASC suppressing activity in vitro

The ASCs were treated with mitomycin C (50 µg/ml, Sigma-Aldrich) for 20 minutes at 37°C, seeded at different concentrations in flat-bottom 96-well plates and left to adhere for 3-4 hours. The splenocytes were labeled with 5 µM CFSE (Sigma-Aldrich) and anti-CD3 (1 µg/ml, BD Pharmingen) was added to the cultures as a mitogenic stimulus of T-cells. The cells were collected on day 3, stained with anti-CD4-APC Abs (BD Biosciences) and analyzed in a FACS Canto II flow cytometer (BD Biosciences). Cell division was analyzed by means of FlowJo software (*Tree Star Inc*, Ashland, Oregon).

### Western Blot

The cells were lysed in RIPA buffer (Sigma) containing protease inhibitors (Sigma Aldrich) at 1 x 10⁶ cells/100 mL. The lysates (20 g/sample) were resolved by polyacrylamide sodium dodecyl sulfate gel electrophoresis (SDS-PAGE; 10% polyacrylamide in reducing conditions) and electrotransferred to nitrocellulose membranes (Bio-Rad Hercules CA.). The membranes were blocked with 5% skimmed milk and tested with anti-LRRC32 (Plato-1; ENZO Life Sciences, Ann Arbor, MI) at a dilution of 1: 500 at 4°C ON, followed by 1 hour of incubation with goat anti-mouse IgG-IRDye680 (dilution of 1:20000; LI-COR Biosciences, Cambridge, United Kingdom). The protein was detected using an Odyssey image scanning system (LI-COR Biosciences).

### Confocal microscopy

For LAP/GARP co-staining, mASCs were collected with EDTA as described above and stained with GARP-PE (YGIC86; eBioscience) and purified anti-mouse LAP/TGF-β1 (TW7-16B4) followed by donkey anti-mouse IgG Alexa488 (Life Technologies). For phospho-Smad2/3 staining, mASCs NT, CTRL-LV, LV#3 and LV#6 were seeded in Lab-Tek®II cc2 8-well chamber slide system (Nunc, Rochester, NY) at 5000 cells/well and cultured in MesenCult supplemented with 0.2% FCS for 2 days. The cells were stained with phospho-Smad2/3 (rabbit anti-mouse/human phospho-Smad2 (Ser465/467)/Smad3 (Ser423/425)); Cell Signaling Technologies, Beverly, MA) followed by goat anti-rabbit Ab Alexa555 (Life Technologies) according to the manufacturer's protocol. The sections were mounted using Slowfade® Gold anti-fade reagent containing DAPI (Life Technologies). Images were captured using a Zeiss LSM 710 confocal microscope (Carl Zeiss, Jena, Germany). The degree of colocalization (Manders' overlap coefficient) was calculated using the ZEN 2010 software (Carl Zeiss).

### Measuring TGF-β1 production by ASCs

The ASCs NT, CTRL, #3 and #6 (20,000 cells/cm²) were cultured for 48 hours and the supernatants were stored at -20°C. The cytokine content was analyzed using Ready-Set-Go ELISA kits for TGF-β1 (eBioscience) according to the manufacturer's instructions. For measuring TGF-β1, complete MesenCult was added to wells free of cells and supernatants from wells containing ASCs were collected and frozen at the same time. All the samples were treated with 1M HCI and neutralized with 1M NaOH according to the manufacturer's description for the purpose of activating TGF-β1. The depicted values were obtained by deducting TGF-β1 levels in the MesenCult medium from the cell culture supernatants.

### Statistical analysis

The statistical analysis was performed using the GraphPad Prism software (GraphPad Software, Inc, La Jolla, CA). All the data is depicted as mean (SEM). Data from various groups was compared using the non-parametric Kruskal-Wallis test followed by the subsequent Dunn's test. Values of *p*<0.05 were considered statistically significant.

### RESULTS

### Mesenchymal stromal cells derived from murine adipose tissue (ASCs) express GARP and LAP/TGF-β1 on the surface thereof

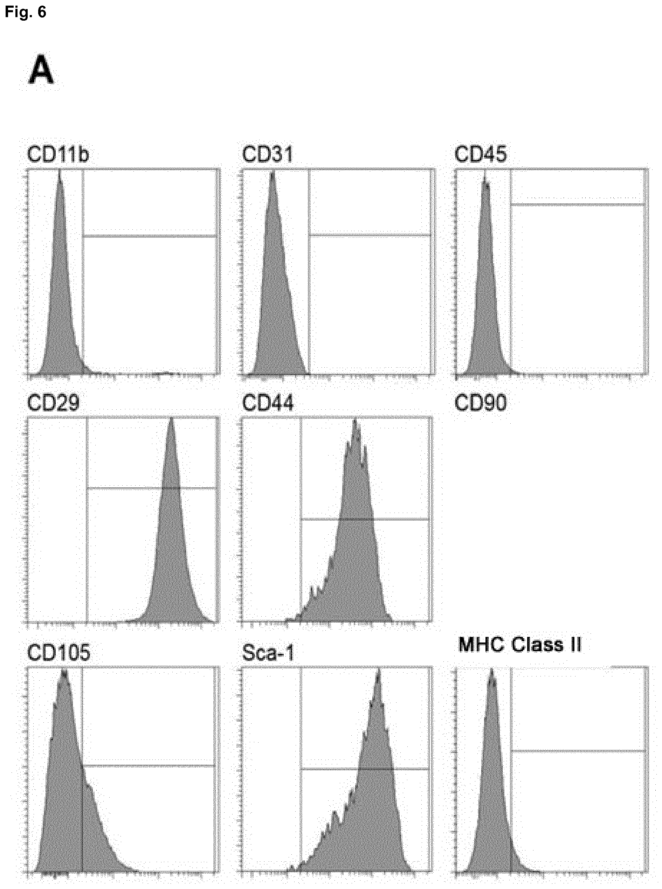

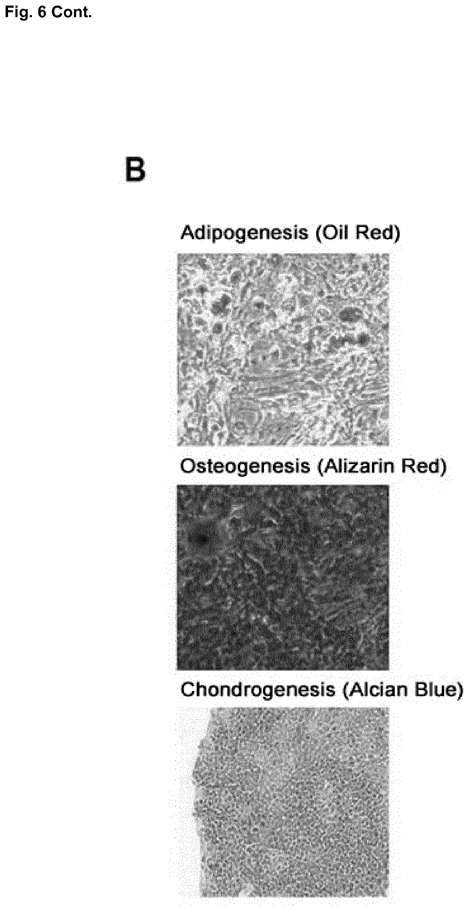

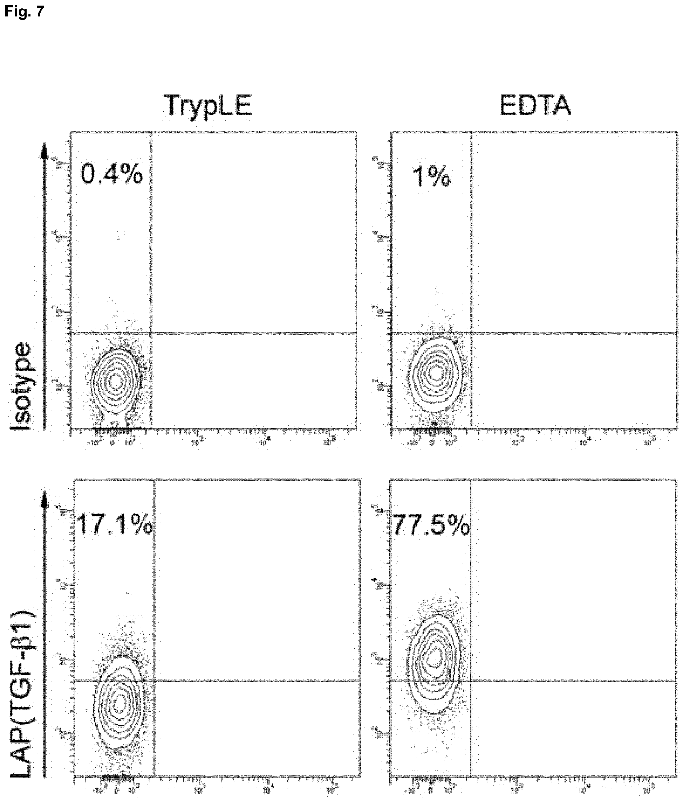

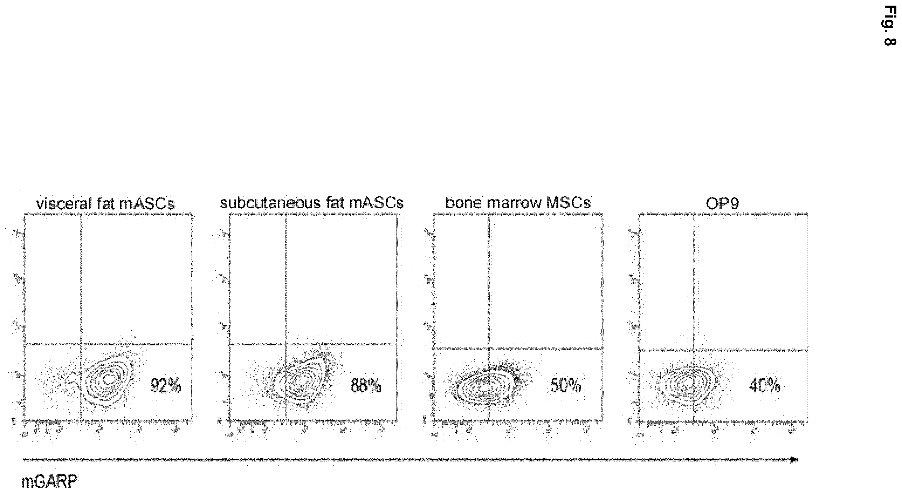

Murine ASCs were isolated from adipose tissue and showed a phenotype and differentiation capacity associated with murine MSC preparations (Figure 6). The authors of the invention have found that mouse and human ASCs expressed LAP/TGF-β1 (mbTGF-β1) on membrane (Figure 1B). The collection of ASCs with TrypLE drastically reduced mbTGF-β1 staining and PBS with 2 mM EDTA was therefore used to separate the ASCs for LAP/TGF-β1 staining (Figure 7). Glycoprotein-A repetitions predominant protein (GARP, also known as LRRC32) has been shown to bind to LAP/TGF-β1 and activated Treg are present (Tran et al., 2009. Proc. Natl. Acad. Sci. 106, 13445-13450). Therefore, GARP mRNA expression in ASCs was evaluated and high mouse and human ASC GARP levels were detected (Figure 1A). The GARP expression in mASCs was 15-20 and 10 times higher compared with the spleen and with OP9 cells, respectively (Figure 1A). This data was confirmed with GARP-specific antibodies using FACS (Figure 1B, Figure 8) and a double staining for GARP and LAP/TGF-β1 showing a clear correlation in the expression intensity of both molecules (Figure 2A). GARP was not expressed in Sca-1+ cells freshly isolated from adipose tissue. However, GARP expression was detected in adhered Sca-1+ cells after culturing *in vitro,* reaching maximum expression level on day 2 (Figure 1C). GARP expression in MSCs was not affected by treatment of TrypLE or type 1 collagenase (results not shown). These results suggest that GARP is not expressed in non-activated MSCs (cells residing in the tissues in non-pathological state) but exclusively when the MSCs acquire the capacity to proliferate and increase their immunomodulatory capacity.

### GARP links LAP/TGF-β1 to the ASC surface

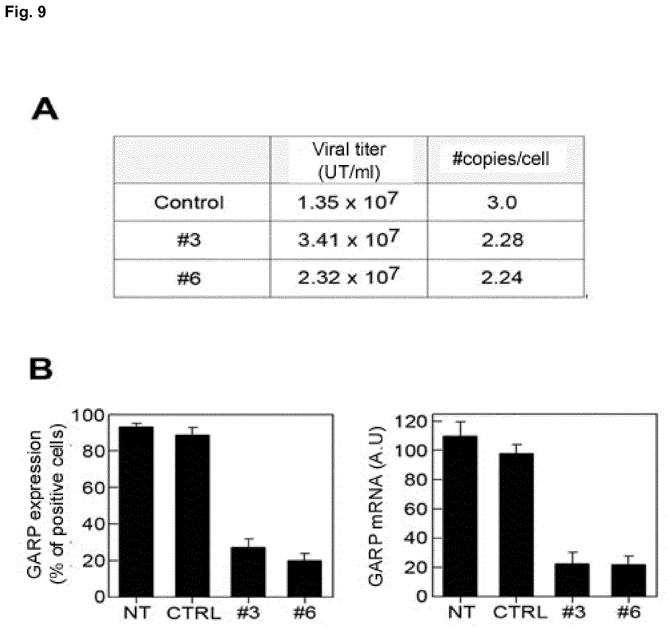

TGF-β can bind to the cell membrane in different ways (Glinka et al., 2008. J. Leukoc. Biol. 84:302-310; Oida et al., 2010. J. Immunol. 185:3529-3535; Yang et al., 2013. PLoS One 8:e59456). For the purpose of evaluating if GARP is responsible for the localization of LAP/TGF-β1 on the plasma membrane in mASCs, GARP expression was silenced using lentiviral vectors encoding specific GARP siRNAs. The ASCs were effectively transduced and three days after transfection, GARP expression both at the level of mRNA and protein were reduced by 80% (Figure 9). It was found that GARP silencing significantly reduced LAP/TGF-β1 expression in mASCs suggesting that GARP is responsible for LAP/TGF-β1 expression on the surface of MSCs (Figure 2B).

### GARP silencing increases TGF-β1 secretion

It has been demonstrated that GARP competes with LTBP1 for binding to LAP/TGF-β1 (Wang et al., 2010. Mol. Biol. Cell 23, 1129-1139) and both genes are expressed in mASCs (Figure 3A). Therefore, TGF-β1 secretion by non-transduced ASCs (NT) or ASCs transduced with a vector encoding an siRNA (CTRL) or two different specific GARP siRNA lentiviral vectors (#3 and #6) was analyzed. It was found that mASCs which GARP has been silenced (both # 3 and # 6) secrete significantly more TGF-β1 compared with mASCs NT and CTRL (Figure 3B). In contrast, TGF-β1 mRNA levels were similar in all the cell populations (Figure 3C). GARP is also involved in TGF-β1 activation regulation, so TGF-β1 levels in activated non-acid supernatants of mASCs NT, CTRL and #3 and #6 were measured. It was found that NT and CTRL did not lead to any TGF-β1 detection, suggesting that the secreted TGF-β1 remains inactive as LAP/TGF-β1. However, in the supernatants of ASCs #3 and #6, low amounts of free active TGF-β1 were detected, suggesting that TGF-β1 regulation is hindered in the absence of GARP (Figure 3D).

### Effect of GARP on mASC proliferation

TGF-β1 has been shown to promote and inhibit cell proliferation depending on cell concentration and cell type. Therefore, the effect of the presence or absence of GARP on mASC proliferation *in vitro* was analyzed. While the proliferation of ASCs transduced with the siRNA control was similar to that of non-transduced cells, ASCs #3 and #6 showed a significant reduction in proliferative capacity (Figure 4A and B).

### Effect of mbTGF-β on the immunomodulatory capacity of ASCs

TGF-β1 membrane ligand has been shown to participate in the suppression of T-cell responses, induction of regulatory T-cells and "natural killer" (NK) cells induced by anergy (Ostroukhova et al., 2006. J. Clin. Invest. 116, 996-1004; Andersson et al., 2008. J. Exp. Med. 205, 1975-1981; Ghiringhelli et al., 2005. J. Exp. Med. 202, 1075-1085). The possible role of mbTGF-β1/GARP of ASC in mediated immunomodulation was to be evaluated. It was found that the GARP silencing significantly reduced the suppressing capacity of ASCs in T-cell proliferation *in vitro* (Figure 5A and B). Ostroukhova *et al.* (2006) demonstrated that regulatory T-cells mbTGF-β1+, but not regulatory T-cells mbTGF-β- in combination with soluble TGF-β1, could induce Notch-HES1 signaling in responder T-cells (Ostroukhova et al., 2006. J. Clin. Invest. 116, 996-1004). However, HES1 expression was not induced in T-cells co-cultured with mASCs and no difference in HES1 expression was detected in T-cells cultured with ASCs, with or without GARP expression, suggesting that mASCs do not control T-cell proliferation through the Notch 1 signaling pathway (data not shown).

### The GARP⁺ fraction of murine bone marrow cells contains most of the CFU-F cells

GARP is not expressed in freshly isolated mASCs but are induced in the culture *in vitro.* In contrast, a small GARP⁺ cell population was found in the bone marrow (0.03%) and by using ASC categorization, the authors of the present invention were capable of increasing the GARP⁺ cell fraction 150-fold. Curiously, when enriched GARP⁻ and GARP⁺ cells from BM were cultured, it was found that the GARP⁺ fraction produced 15-fold more CFU-F compared with GARP⁻ cells from BM.

The authors of the present invention have described for the first time GARP expression on the surface of human and murine MSCs. They have demonstrated that the presence of GARP in the MSCs is important in three aspects:
(1) as a TGF-β1 secretion and activation regulator;
(2) for the presentation of TGF-β1 on the surface of the MSCs, which could induce a response different from that of secreted TGF-β or TGF-β bound to ECM;
(3) Finally, since GARP expression is limited to foxp3⁺ Treg, megakaryocytes and platelets, a simple combination of GARP with one or two other surface antigens (for example, negative for CD45 and positive for Sca-1/CD44 (mice) or CD44/CD73 (human) may be sufficient for identifying MSCs *in vitro* and *in vivo.*

Furthermore, the examples have demonstrated that GARP silencing in mASCs using siRNA encoding lentiviral vectors reduced mbTGF-β1, while causing an increase in LAP/TGF-β1 secretion, in accordance with the study conducted by Wang *et al.* in HEK293 and 293T cells (Wang et al., 2010. Mol. Biol. Cell 23, 1129-1139). GARP^{-/low} ASCs showed a significant reduction in their proliferative capacity. The authors of the present invention propose that this phenotype does not depend of the toxic effects of the siRNAs since the proliferation of control cells containing many vectors in the cell genome (Figure 9) was not affected. Given that TGF-β1 could inhibit mASC proliferation *in vitro,* the inventors propose that the increase in TGF-β1 secretion (and activation) by GARP^{-/low} cells could explain the deterioration thereof in proliferation. However, the addition of large amounts of neutralizing antibody TGF-β1/2/3 did not increase GARP^{-/low} cell proliferation. Recently, Zhou *et al.* showed a positive correlation between GARP expression levels in T-cells and HeLa cells and their proliferative capacity (Zhou et al., 2013. J. Immunol. 190, 6579-6588). Furthermore, several reports have detected that the amplification of chromosomal fragments 11q13, containing the GARP gene, in solid tumors, also suggests that GARP can control cell proliferation, this is in accordance with data obtained by the inventor (Bekri et al., 1997. Cytogenet Cell Genet. 79, 125-131; Maire et al., 2003. Genes Chromosomes Cancer 37, 389-395). However, the mechanisms underlying the suppression of the proliferation of cells lacking GARP are unknown.

The GARP-mediated presentation of TGF-β1 on the surface of activated regulatory T-cells is important for tolerance to infection and for T-cell differentiation but also contributes to the suppressing capacity of these cells. The inventors have demonstrated that GARP expression in mASCs also contributes to their capacity to inhibit T-cell responses *in vitro.* The GARP⁺ ASCs are two times more effective than GARP^{-/low}. This is an interesting finding taking into account the large number of immunoregulatory molecules expressed by the MSCs, including iNOS, PGE₂ and growth factors (Shi et al., 2011. Clin. Exp. Immunol. 164, 1-8). It is unknown how the presence of GARP increases the suppressing capacity of mASCs and how the secretion of TGF-β may depend on the regulation thereof but rather and also on the participation of mbTGF-β. It is important to point out that many research works demonstrate that cell response to TGF-β can differ depending on if TGF-β is secreted or is presented on the membrane (Han et al., 2009. J. Immunol. 182, 111-120; Ostroukhova et al., 2006. J. Clin. Invest. 116, 996-1004; Zhang et al., 2011. Hepatology 53, 306-316). The reason for these differences is not well understood, but it is believed that mbTGF-β, through the cell-cell contact, can give rise to (i) a high local concentration of active TGF-β, (ii) sustained TGF-β and (iii) allow other interactions to take place modulating the response to TGF-β in a manner in which the secreted TGF-β cannot be imitated (Ostroukhova et al., 2006. J. Clin. Invest. 116, 996-1004; Zhang et al., 2011. Hepatology 53, 306-316). As mentioned above, mbTGF-β1, but not the secreted TGF-β1, induces Notch1/HES1 signaling in target T-cells inhibiting their proliferation (Ostroukhova et al., 2006. J. Clin. Invest. 116, 996-1004). The inventors were not able to detect any difference in HES1 induction in T-cells co-cultured with any of ASCs NT or GARP^{-/low}ASCs. However, it has been shown that the ASCs express lower levels of Notch1 ligands compared with BM-MSCs and these cells may not be capable to induce Notch1/HES1 signaling in target T-cells.

Recent studies have demonstrated that MSCs have more in common with pericytes, including the perivascular localization, the *in vitro* morphology, the surface markers and the differentiation capacity (Nombela-Arrieta et al., 2011. Nat. Rev. Mol. Cell Biol. 12, 126-131). A model in which the MSCs are cells resting in a perivascular location which become active after injury and therefore adopt the described MSC phenotype *in vitro* has been proposed (Caplan et al., 2011. Cell Stem Cell 9, 11-15).

In summary, it has been demonstrated for the first time that mouse and human MSCs, particularly ASCs, express mbTGF-β1 presented by GARP. GARP regulates TGF-β1 secretion and controls both ASC proliferation and immunomodulatory capacity. GARP shows a limited expression profiled compared with most MSC-associated antigens used for characterization thereof, including CD44, CD90 and CD105. In this sense, GARP may aid in the identification of activated MSCs *in vitro* and *in vivo.*

## Claims

1. An *in vitro* method for the identification and/or isolation of an activated mesenchymal stem cell (MSC) from a biological sample, comprising the detection of the GARP protein on the surface of said cell and the isolation of said MSCs based on the expression of said protein on the surface thereof.

2. Use of the GARP surface protein as a marker of an activated mesenchymal stem cell *in vitro.*

## Patentansprüche

1. *In vitro*-Verfahren zur Identifizierung und/oder Isolierung einer aktivierten mesenchymalen Stammzelle (MSC) aus einer biologischen Probe, umfassend die Erfassung des GARP-Proteins auf der Oberfläche der genannten Zelle und die Isolierung der genannten MSC basierend auf die Expression des genannten Proteins auf der Oberfläche derselben.

2. Verwendung des GARP-Oberflächenproteins als Marker einer aktivierten mesenchymalen Stammzelle *in vitro.*

## Revendications

1. Procédé in vitro d'identification et/ou de séparation d'une cellule souche mésenchymateuse activée (MSC) à partir d'un échantillon biologique, comprenant la détection de la protéine GARP à la surface de ladite cellule et la séparation desdites MSC basées sur l'expression de ladite protéine à la surface de celle-ci.

2. Utilisation de la protéine de surface GARP comme marqueur d'une cellule souche mésenchymateuse activée in vitro.
